# EUROPÄISCHE PATENTSCHRIFT

(11) **EP 1 282 819 B1**
(45) Veröffentlichungstag und Bekanntmachung des Hinweises auf die Patenterteilung: **26.11.2008**
(21) Anmeldenummer: 01925266.7
(22) Anmeldetag: 08.05.2001
(51) Int. Cl.: G01N 33/543, C12Q 1/68, B01J 19/00, C12Q 1/00, G01N 33/545

(54) **VERFAHREN ZUR IMMOBILISIERUNG VON ERKENNUNGSKOMPONENTEN**
METHOD FOR IMMOBILIZING RECOGNITION COMPONENTS
PROCEDE D'IMMOBILISATION DE CONSTITUANTS DE RECONNAISSANCE

(30) Priorität: 10.05.2000 DE 10022750
(43) Veröffentlichungstag der Anmeldung: 12.02.2003
(73) Patentinhaber: ASULAB S.A., 2074 Marin (CH)
(72) Erfinder: SCHUHMANN, Wolfgang, 44799 Bochum (DE)
(74) Vertreter: Thérond, Gérard Raymond
(86) Internationale Anmeldenummer: PCT/CH2001/000279
(87) Internationale Veröffentlichungsnummer: WO 2001/086298

(56) Entgegenhaltungen:
- EP-A- 0 601 720
- WO-A-00/77523
- WO-A-98/52042
- US-A- 5 849 174
- HABERMUELLER KATJA ET AL: "A low-volume electrochemical cell for the deposition of conducting polymers and entrapment of enzymes." ELECTROANALYSIS, Bd. 10, Nr. 18, Dezember 1998 (1998-12), Seiten 1281-1287, XP001016361 ISSN: 1040-0397

## Beschreibung

Die Erfindung betrifft ein neuartiges einfaches Verfahren zur Immobilisierung von molekularen Funktionselementen, insbesondere von biologischen Erkennungselementen auf gegebenenfalls leitenden Oberflächen, und die mit diesem Verfahren erhältlichen Gegenstände.

Die Immobilisierung von Erkennungselementen, insbesondere biologischen und biochemischen Erkennungselementen, auf gegebenenfalls leitenden Oberflächen ermöglicht eine Vielzahl unterschiedlicher Anwendungen im Bereich der Sensorik, insbesondere der Biosensorik, der chemischen oder biochemischen Kombinatorik und der medizinischen Diagnostik, beispielsweise für ein Screening. Dabei kann die Immobilisierung der Erkennungselemente beispielsweise durch Absorption wie in US-A-3 839 175 offenbart, Vernetzung, D.J. Strike, N.F. de Rooij, M. Koudelka-Hep, Biosens. Bioelectron., 10 (1995) 61-66, oder durch Einschluß in einen Polymerfilm, wie bei W. Schuhmann, Mikrochim. Acta, 121 (1995) 1-29, offenbart, erfolgen. Bei der für die Immobilisierung der Erkennungselemente verwendeten Matrix handelt es sich meist um ein Polymer, das durch radikalische Polymerisation auf der gegebenenfalls leitenden Oberfläche abgeschieden wird. Die radikalische Reaktion kann dabei elektrochemisch, chemisch oder auch photochemisch, vgl. z.B. EP-A-0 691 408, induziert werden. Ein Nachteil dieser bekannten radikalischen Polymerisationen ist die Wirkung von Sauerstoff als Inhibitor einer solchen Reaktion. Aufgrund dieser Wirkung muß die gesamte Reaktion unter einer Schutzgasatmosphäre stattfinden. Weitere Verfahren zur Immobilisierung sind lithographische Verfahren auf Festkörpern, wie in G.H. McGall, A.D. Barone, M. Diggelmann, S.P.A. Fodor, E. Gentalen, N. Ngo, J. Am. Chem. Soc., 119 (1997) 5081, offenbart, und das Druckverfahren, wie von G.F. Khan, Electroanalysis, 9 (1997) 325-329, offenbart. Beide Verfahren erfordern einen hohen materiellen Aufwand und sind nur zum Teil für eine lokale Abscheidung auf gegebenenfalls leitenden Oberflächen bei hoher Auflösung (von < 100 µm) geeignet.

Der Erfindung liegt die Aufgabe zugrunde, Beschichtungen von Sensoren in Form einer funktionalisierten, insbesondere enzymatisch aktiven Elektrodenoberfläche bereitzustellen, wobei u.a. folgende Ziele zu erreichen sind: schnelle, einfache und kostengünstige Herstellung; Immobilisierung auf unterschiedlichsten gegebenenfalls leitenden Oberflächen, z.B. üblichen Elektrodenmaterialien; Ausschluß von Sauerstoff bei der Filmherstellung ist nicht notwendig; die Filmdicke kann zwanglos variiert werden; modifizierte oder unmodifizierte Komponenten, beispielsweise Latexkügelchen, Glaskügelchen, Graphitteilchen usw., können in einfacher Weise von der Schicht umschlossen werden, es können mehrschichtige Filme mit unterschiedlichen Funktionen aufgebaut werden; lokale Abscheidungen sind mit örtlich begrenzten pH-Gradienten möglich und damit der Aufbau von Arraystrukturen, z.B. für die kombinatorische Chemie, Biosensorarrays, Immunoassays oder unterschiedliche Screeningverfahren; Möglichkeit der Oberflächenmodifizierung; Möglichkeit der örtlichen Auflösung des Films; sowie Möglichkeit des Einsatzes von Fluoreszenzmarkern oder anderen fluoreszierenden Erkennungskomponenten. Die Aufgabe wurde durch die Gegenstände der vorliegenden Ansprüche gelöst.

Das Verfahren der Elektrodepositionslackierung (EPD), F. Beck, Electrochim. Acta, 33 (1988) 839-850, wird zum Korrosionsschutz bei Autokarosserien und Zubehörteilen, Heizkörpern sowie Getränkedosen industriell angewandt. Bei der anodischen Elektrodeposition werden die durch die Wasserzersetzung an der Anode freigesetzten Protonen dazu benutzt, ein lösliches, aufgrund von Carboxylatgruppen negativ geladenes Polymer zu neutralisieren und damitauszufällen. Die diesem Verfahren zugrundeliegenden Vorgänge können wie nachstehend zusammengefaßt werden:
- Wasserzersetzung:: 2 H₂O → 4 H⁺ + O₂ + 4 e⁻
- Polymerabscheidung::

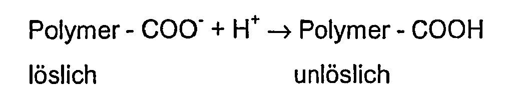

Anschließend wird der Film gewöhnlich durch Erhitzen (in der Regel 165-185°C) auf der Oberfläche zu einer undurchlässigen Beschichtung vernetzt, die eine größere mechanische sowie chemische Beständigkeit aufweist. Neben dem sogenannten anodischen Elektrodepositionslack (AED) gibt es den kathodischen Elektrodepositionslack (KED). Dieser wird durch lokal erzeugte Hydroxidionen auf der gegebenenfalls leitenden Oberfläche abgeschieden. Diesem Vorgang liegt nachstehendes allgemeines Reaktionsschema zugrunde:
1. 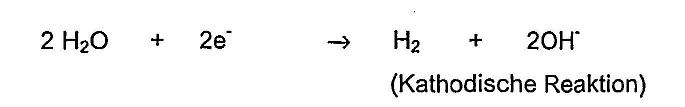
2.

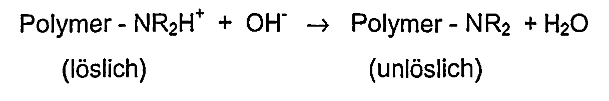

Eine weitere Gruppe von Verbindungen sind jene amphoterer Natur, z.B. Peptide, Oligopeptide oder Proteine. Durch geeignete Wahl der Elektrodenreaktion kann der mit sowohl positiv als auch negativ geladenen Gruppen in unterschiedlichen Verhältnissen versehene Stoff, z.B. das Peptid, insbesondere ein Oligopeptid oder Polypeptid, an der Elektrodenoberfläche in seinem unlöslichen Zustand (isoelektrischer Punkt) abgeschieden werden.

Das Resultat der Elektrodenreaktion, nämlich ein abgeschiedener, im wesentlichen organischer Film, kann gegebenenfalls weiterbehandelt werden, indem Modifizierungen physikalische (z.B. Wärme) oder chemische (z.B. chemische Derivatisierung) vorgenommen werden können.

US5,849,174 offenbart ein Verfahren zur Herstellung eines Glucose-Biosensors, dadurch gekennzeichnet, daß eine Glucose Oxidase enthaltende Polyanyline-Schicht durch elektrochemische Polymerisation auf eine Elektrode abgeschieden wird.

In der vorliegenden Erfindung wird somit ein Stoff mit Erkennungsfunktion, z.B. ein Enzym sowie Harzteilchen in Form einer Harzemulsion, als Elektrolyseflüssigkeit vorgelegt und aufgrund der Erzeugung von Protonen (Hydroniumionen) oder Hydroxylionen an der Oberfläche der Zielelektrode werden Harzabscheidungen erzeugt, die z.B. in der Elektrolytlösung gleichzeitig vorliegende Enzymmoleküle einschließen und so immobilisieren. Aufgrund der Tatsache, daß dieser Vorgang elektrochemisch erfolgt, können Elektroabscheidungen beliebiger Form in einem beliebigen Muster z.B. auf einer relativ großflächigen Zielelektrode abgeschieden werden.

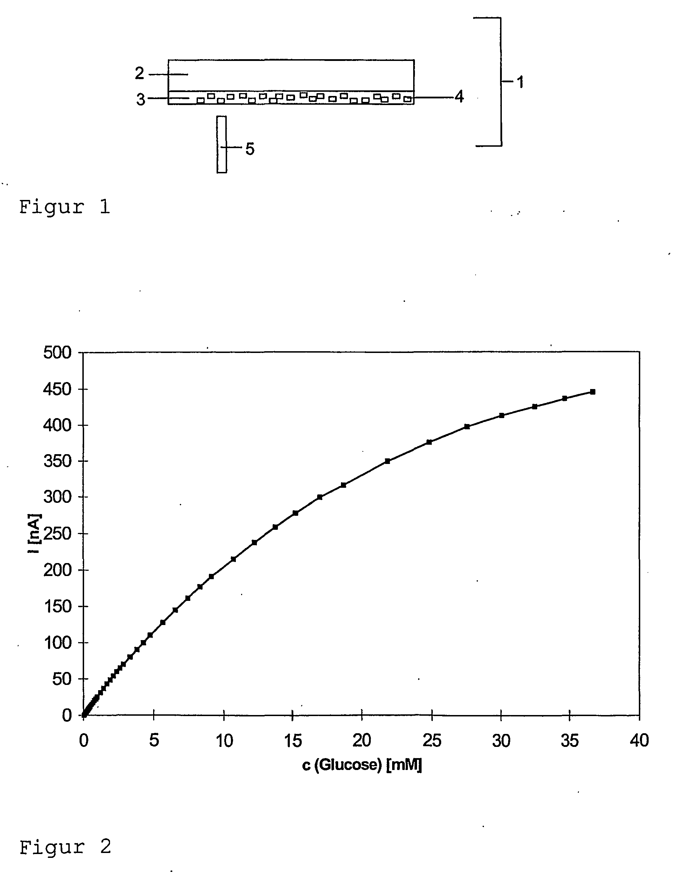

Figur 1 erläutert den prinzipiellen Aufbau einer durch das erfindungsgemäße Verfahren erhaltenen mit Erkennungselementen funktionalisierten Fläche 1. In Figur 1 gibt Bezugsziffer 1 das durch das Verfahren erhaltene Produkt, nämlich die mit Erkennungselementen funktionalisierte Fläche, wieder. Bezugsziffer 2 gibt den gegebenenfalls elektrisch leitfähigen Körper wieder. Bezugsziffer 3 veranschaulicht die abgeschiedene Lackschicht, die den Stoff 4 mit Erkennungsfunk-tion einschließt. Sondenspitze 5 soll verdeutlichen, daß dieses Verfahren in einem Feld zwischen Sonde 5 und Körper 2 sehr gezielt vorgenommen werden kann.
Figur 2 zeigt die Aufnahme einer Kalibrierkurve nach Beispiel 1, nämlich die Kalibrierkurve einer mit GOD (Glucoseoxidase) modifizierten Platinscheibenelektrode (∅ 1 mm). Die Messung erfolgte in 20 ml Phosphatpuffer, pH 7, unter Zugabe von 0,1 mM Glucoselösung. Das an der beschichteten Elektrode angelegte Potential betrug dabei 600 mV gegen Ag/AgCl.

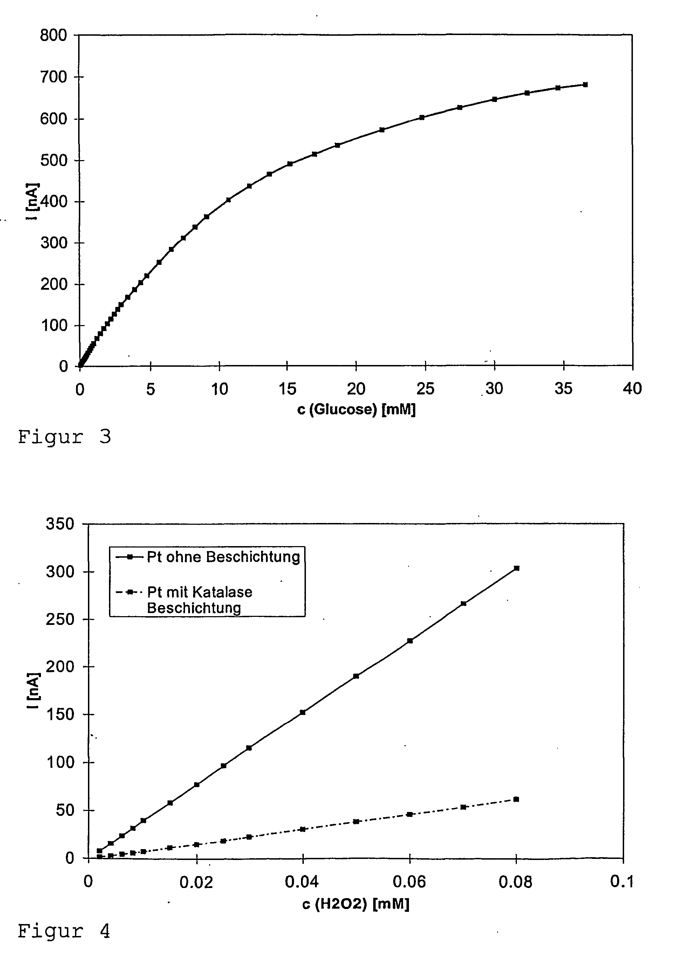

Figur 3 zeigt die Kalibrierkurve einer mit GOD modifizierten, mittels Dickschichttechnik hergestellten Kohlenstoffelektrode (Grundstruktur des Sensors ist von der Firma SensLab). Die Messung erfolgte in 20 ml Phosphatpuffer, pH 7, unter Zugabe von 0,1 mM Glucoselösung. Das an der beschichteten Elektrode angelegte Potential betrug dabei 600 mV gegen Ag/AgCl.
Figur 4 zeigt den Vergleich der Wasserstoffperoxid-Sensitivität einer mit Katalase modifizierten Elektrode zu einer unmodifizierten Platinscheibenelektrode (∅ 1 mm). Die Messung erfolgte in 20 ml Phosphatpuffer, pH 7, unter Zugabe von 2 mM Wasserstoffperoxidlösung. Das an den Elektroden angelegte Potential betrug dabei 600 mV gegen Ag/AgCl.

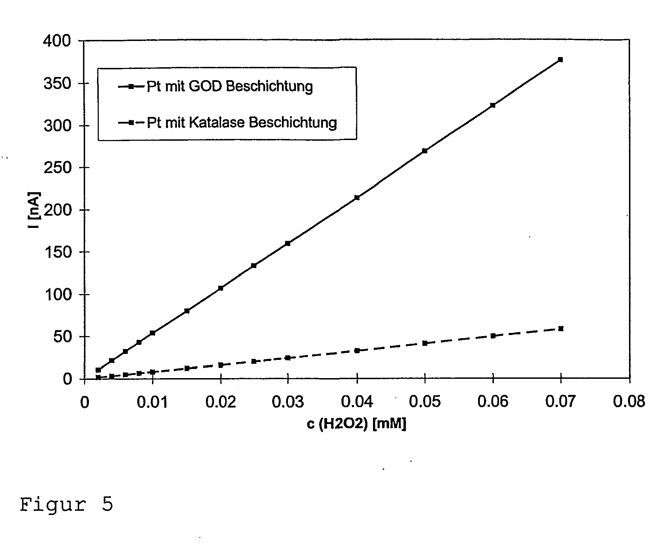

Figur 5 zeigt den Vergleich der Wasserstoffperoxid-Sensitivität einer mit Katalase modifizierten Elektrode zu einer GOD-modifizierten Platinscheibenelektrode (∅ 1 mm). Die Messung erfolgte in 20 ml Phosphatpuffer, pH 7, unter Zugabe von 2 mM Wasserstoffperoxidlösung. Das an den Elektroden angelegte Potential betrug dabei 600 mV gegen Ag/AgCl.

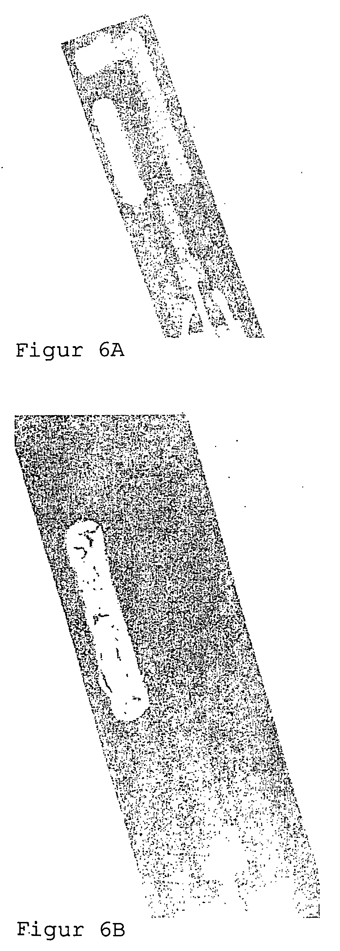

Figuren 6A und 6B zeigen eine Mikroskopaufnahme eines modifizierten Platinmikroarrays. Auf dieser Struktur sind zwei unterschiedliche Filme abgeschieden. Die L-förmige Bandelektrode wurde mit einem GOD-Film beschichtet, während die kleinere, gerade Bandelektrode mit einem Bead-Film versehen wurde. Die obige Aufnahme zeigt die Struktur bei Normallicht, die untere bei Fluoreszenzlicht.

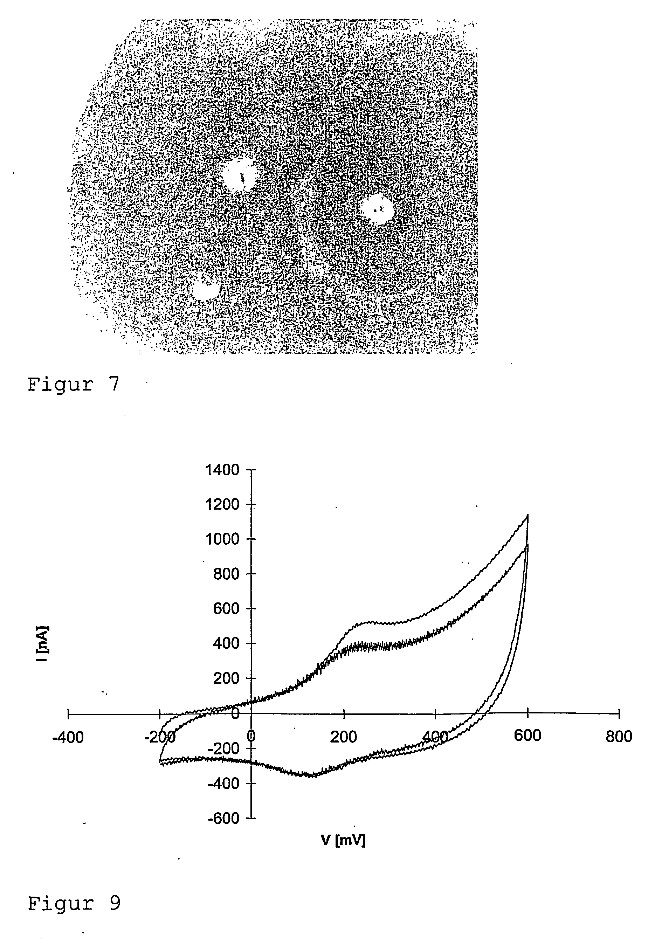

Figur 7 zeigt eine Mikroskopaufnahme dreier mittels eines SECM lokal auf einer Goldscheibenelektrode (∅ 3 mm) abgeschiedener Polymerpunkte.

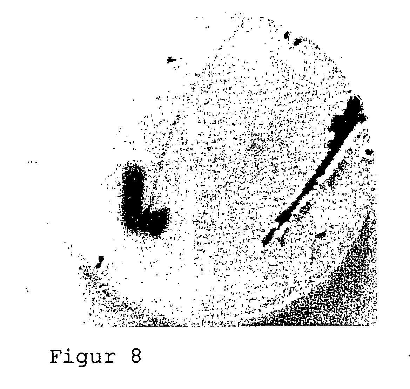

Figur 8 zeigt eine Mikroskopaufnahme eines mittels SECM lokal aufgelösten (L-Form) Polymerfilms. Für die Auflösung wurde mehrfach ein Potentialpuls von -2000 mV gegen Ag/AgCl für jeweils 1 s an der lateral über die Oberfläche bewegten Mikroelektrode angelegt. Die Auflösung erfolgte in 5 mM Rutheniumhexamminlösung.
Figur 9 zeigt ein Cyclovoltammogramm (CV) einer mit redoxaktiven Gruppen ([Os(bpy)₂(Histamin)Cl]Cl) modifizierten Platinscheibenelektrode (∅ 1 mm). Die Messung erfolgte von -200 bis +600 mV gegen Ag/AgCl in einer 0,1 M Lithiumperchlorat-Lösung.

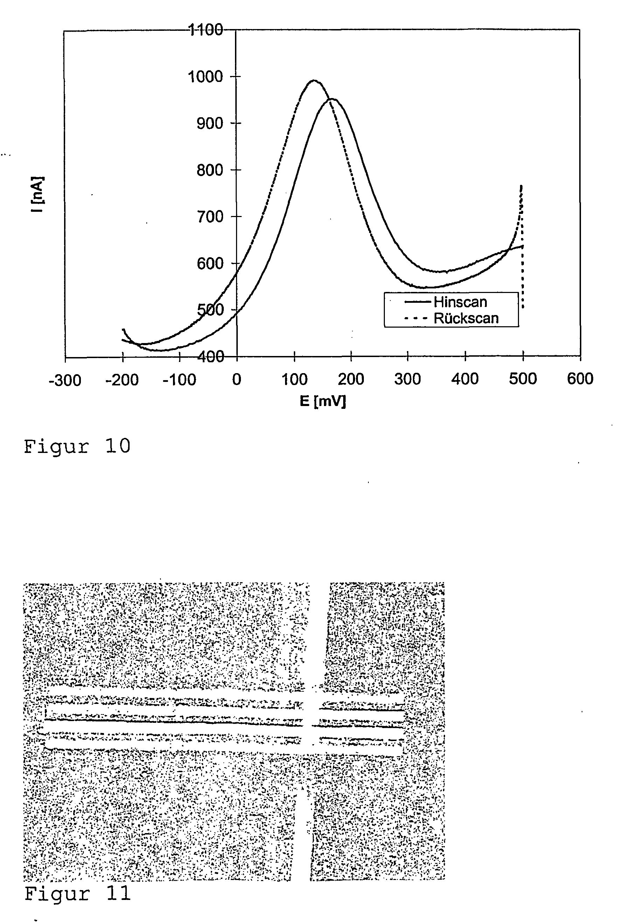

Figur 10 zeigt ein Differential-Puls-Voltammogramm (DPV) einer mit redoxaktiven Gruppen ([Os(bpy)₂(Histamin)Cl]Cl) modifizierten Platinscheibenelektrode (∅ 1 mm). Die Messung erfolgte von -200 bis +600 mV gegen Ag/AgCl in einer 0,1 M Lithiumperchlorat-Lösung.
Figur 11 zeigt eine Array-Anordnung, wobei die zweite Bande beschichtet wurde.

Als gegebenenfalls elektrisch leitfähiger Körper 2 können beliebige Materialien verwendet werden, die in der Lage sind, zu einer Elektrode, beispielsweise Elektrode 5, ein Potential (zur Erzeugung von beispielsweise H+ oder OH-) und ein Feld (gegebenenfalls zur Wanderung der Teilchen) aufzubauen, das die Emulsion auf der Oberfläche von 2 abscheiden kann. Geeignet sind hierfür insbesondere alle Edelmetalloberflächen, vor allem Oberflächen von Gold, Silber, Platin, Palladium, Iridium, Rhenium, Quecksilber, Ruthenium und Osmium. Des weiteren sind sich elektrochemisch weitestgehend inert verhaltende Metalle, wie Chrom, Nickel, Kobalt, Eisen und deren als "Edelstahl" bekannte Legierungen sowie Titan-, Zirkonium- und Hafnium-Oberflächen, geeignet. Außerdem sind auch bestimmte passivierte Metalle oder Legierungen, z.B. Aluminium, Gallium oder Aluminiumlegierungen, die sich während des elektrochemischen Vorgangs inert verhalten, geeignet. Grundsätzlich dürfen die Metalle die eingesetzten Erkennungselemente, z.B. Enzyme, nicht hemmen. Die Größe einer Zielelektrode ist vom angestrebten Zweck abhängig. In der Regel betragen die Flächen von 0,5 - 50 mm². Bei entsprechender Dimensionierung ist es jedoch durchaus möglich, diesen Bereich um das 3-, 5-, 10-, 20-, 100-, 250- oder 500fache zu vergrößern oder zu verkleinern. Eine Elektrodenoberfläche wird zweckmäßigerweise vor dem Gebrauch poliert und zusätzlich behandelt, beispielsweise durch Platinisieren.

Entscheidend ist, daß die Elektrodenoberfläche so beschaffen ist, daß die zur Abscheidung erforderlichen elektrochemischen und/oder elektrostatischen Vorgänge vonstatten gehen können.

Es ist auch möglich, Halbleiterelektroden, wie Siliziumoberflächen, GaAs, ITO, In₂O₃ u.ä., die erforderlichenfalls mit entsprechenden Elementspuren dotiert sind, bzw. Kohlenstoffelektroden, bei entsprechender Dimensionierung und Oberflächenbehandlung zu verwenden. Außerdem ist es möglich, beliebige Oberflächen, die nicht metallischer Art sind, einzusetzen, sofern ihnen ein elektrisches Potential auferlegt werden kann, das ausreicht, um das Harz aus der Emulsion auf ihnen abzuscheiden.

Als Harzemulsion 3 können beliebige bekannte Harzemulsionen eingesetzt werden, die für eine Elektrodeposition geeignet sind. Hierzu gehören organische Verbindungen höheren Molekulargewichts im Bereich von 300 bis 50000; 600 bis 30000; 1000 bis 20000; 800 bis 10000; 1000 bis 15000, aber sofern löslich bzw. emulgierbar auch höheren Molekulargewichts von den vorstehend ausgegebenen Werten bis zu 50000, 70000, 100000, 250000 oder auch 1000000, insbesondere Harze mit elektrischen Ladungen ionischer Natur, die durch funktionelle Gruppen an den sie ausmachenden Monomeren erzeugt werden. Beispiele sind modifizierte Polystyrole, Olefine, Polyamide und Vinyl- bzw. Acrylverbindungen, aber auch biochemisch interessante Polymere, wie Proteine, Polysaccharide mit funktionellen Gruppen, Pflanzengummen, Oligo- und Polynukleotide. Insbesondere haben sich α,β-olefinisch ungesättigte Carbonsäurepolymere für die anodische Abscheidung bewährt.

Ein bevorzugtes Harz für die in der Erfindung verwendete Harzemulsion zur anodischen Elektrodeposition ist z.B. eine Harzzubereitung auf der Basis eines Carboxylgruppen, verkappte Isocyanatgruppen, Hydroxyl- und Ethergruppen enthaltenden Copolymerisats, das durch zumindest teilweise Salzbildung mit Ammoniak oder einer organischen Base wasserlöslich oder in Wasser dispergierbar ist, wobei das Copolymerisat einpolymerisiert enthält:
1. mindestens eine α,β-olefinisch ungesättigte Carbonsäure mit 3 bis 5 Kohlenstoffatomen oder einen Halbester einer α,β-olefinisch ungesättigten, 3 bis 5 Kohlenstoffatome enthaltenden Dicarbonsäure,
2. 10 bis 35 Gewichtsprozent eines mit CH-, OH- oder NH-aktiven Verkappungsmitteln verkappten N(1-Alkenyl)isocyanats,
3. 20 bis 50 Gewichtsprozent eines Addukts aus einem Epoxidharz auf der Basis von Bisphenol A und Epichlorhydrin mit einem Molekulargewicht zwischen 380 und 3500 und einem olefinisch ungesättigten, 3 bis 20 Kohlenstoffatome enthaltenden Alkohol,
4. 5 bis 64 Gewichtsprozent einer oder mehrerer unter 1. bis 3. nicht genannter copolymerisierbarer olefinisch ungesättigter Verbindungen,
mit der Maßgabe, daß das Copolymerisat ein mittleres Molekulargewicht zwischen 1000 und 20000 aufweist, die Komponente (1) in einer solchen Menge einpolymerisiert enthält, daß die Säurezahl des Copolymerisats 35 bis 150 mg KOH/g beträgt und die Summe der unter 1 bis 4 genannten Prozentzahlen 100 ist.

Das vorstehend erwähnte Copolymerisat weist ein mittleres Molekulargewicht zwischen 1000 und 20000 auf. Die Komponente wird in einer solchen Menge einpolymerisiert, daß die Säurezahl des Copolymerisats 35 bis 150 mg KOH/g beträgt. Die Summe der unter 1 bis 4 genannten Prozentzahlen beträgt 100. Hervorzuheben ist, daß das Äquivalentverhältnis der reaktiven Wasserstoffatome der Komponente (3) zu den verkappten Isocyanatgruppen im Copolymerisat vorzugsweise etwa 1:1 beträgt. Des weiteren wird als Komponente (2) ein Addukt aus Vinylisocyanat oder Propenylisocyanat und Cyclohexanol, tert-Butanol, Triazabenzol oder ε-Caprolactam im Molverhältnis Isocyanat/Verkappungsmittel von vorzugsweise 1:1 verwendet. Insbesondere enthält das Copolymerisat als Komponente (1) Acryl- oder Methacrylsäure, als Komponente (2) mit ε-Caprolactam verkapptes Vinylisocyanat, als Komponente (3) ein Umsetzungsprodukt eines Epoxidharzes aus Bisphenol A und Epichlorhydrin mit einem mittleren Molekulargewicht von etwa 900 und Allylalkohol sowie als Komponente (4) 2-Ethylhexylacrylat oder Butylacrylat einpolymerisiert.

Bevorzugt ist auch eine harzartige Masse, die als Gemisch eines wasserlöslichen harzartigen Materials und eines wasserunlöslichen harzartigen Materials eingesetzt wird. Das Gemisch wird in einem wässerigen Medium, von dem Wasser der Hauptbestandteil ist, dispergiert. Die wasserlöslichen harzartigen Materialien sind polymer und werden durch Einbau ausreichend hydrophiler Gruppen in das Polymer wasserlöslich gemacht. Die hydrophilen Gruppen können ionische Salzgruppen sein, beispielsweise anionische Salzgruppen, wie Carbonsäure- und Sulfonsäuresalzgruppen, oder kationische Salzgruppen, wie Aminsalzgruppen und quaternäre Ammoniumsalzgruppen. Die bevorzugten hydrophilen Gruppen sind anionische Gruppen, besonders bevorzugt sind es Salze von Carbonsäuregruppen. Gewöhnlich wird ein Polymer mit Carbonsäuregruppen hergestellt und dann mit einer wasserlöslichen basischen Verbindung, wie einem organischen Amin oder ein Alkalimetallhydroxid, neutralisiert.

Der Begriff "wasserlöslich" bedeutet in diesem Zusammenhang, daß das harzartige Material, in Wasser löslich gemacht, bei einem Harzfeststoffanteil von bis zu 25%, gewöhnlich 1 bis 20 Gewichtsprozent, ohne Hilfe eines von außen zugegebenen Tensids dispergiert werden kann. Die Lösung oder Dispersionen erscheinen häufig optisch durchsichtig oder durchscheinend, wobei das Harz in der dispergierten Phase vorliegt und eine mittlere Teilchengröße von 0,12 und weniger, gewöhnlich weniger als 0,03 µm, aufweist. Die mittlere Teilchengröße der wasserlöslichen harzartigen Materialien kann durch Lichtstreuverfahren ermittelt werden.

Die bevorzugten niedermolekularen, wasserlöslichen Polymere sind Acrylcopolymere, die eine anionische Ladung enthalten, vorzugsweise eine Carbonsäuresalzgruppe und besonders bevorzugt eine mit organischem Amin neutralisierte Carbonsäuregruppe.

Unter den niedermolekularen Acrylcopolymeren sind Polymere, hergestellt durch Copolymerisation einer α,β-ethylenisch ungesättigten Carbonsäure mit einem Methacrylsäureester und/oder Acrylsäureester, und im allgemeinen sind Acrylpolymere geeignet, die als Hauptanteil einen Methacrylatester von einem C₁-C₈-Alkohol und einen geringen Anteil eines Acrylatesters mit einem C₁-C₈-Alkohol aufweisen. Die nachstehenden Verbindungen sind übliche Methacrylatester und Acrylatester: Ethylacrylat, Propylacrylat, Isopropylacrylat, Butylacrylat, Isobutylacrylat, sekundäres Butylacrylat, Hexylacrylat, 2-Ethylhexylacrylat, Octylacrylat, Methylmethacrylat, Propylmethacrylat, Isobutylmethacrylat, Butylmethacrylat, sekundäres Butylmethacrylat und tertiäres Butylmethacrylat.

Die verwendeten Acrylpolymere enthalten 0,1 bis 20 Gewichtsprozent einer polymerisierten α,β-ethylenisch ungesättigten Carbonsäureeinheit. Verwendbare α,β-ethylenisch ungesättigte Carbonsäuremonomere sind Methacrylsäure, Acrylsäure, Itaconsäure, Ethacrylsäure, Propylacrylsäure, Isopropylacrylsäure und Homologe dieser Säuren. Methacrylsäure und Acrylsäure sind bevorzugt. Der Prozentsatz an Säure wird so eingestellt, daß die erforderliche Säurezahl in dem Acrylpolymer erzeugt wird. Gewöhnlich sollte die Säurezahl der Acrylpolymere so eingestellt werden, daß sie etwa 30 bis 100 auf die Harzfeststoffanteile beträgt. Die zahlenmittleren Molekulargewichte der wasserlöslichen Acrylpolymere liegen vorzugsweise im Bereich von 10 000 bis 30 000.

Die Acrylpolymere können auch Hydroxylseitengruppen enthalten, was durch Copolymerisation von Hydroxyalkylacrylaten oder Hydroxyalkylmethacrylaten mit den vorstehend genannten Acrylestern erreicht wird. Die Hydroxylgruppen-Seitengruppen liefern Stellen zur anschließenden Härtung, wie mit einem Aminoplast oder einem blockierten Isocyanat. Vorzugsweise sind 5 bis 15 Gewichtsprozent des verwendeten Acrylpolymers von einem Hydroxyalkylacrylat- oder - methacrylatester. Im allgemeinen verwendbare Hydroxyalkylacrylate und - methacrylate enthalten 1 bis 8 Kohlenstoffatome in der Alkylgruppe und sind beispielsweise Hydroxyethylacrylat, Hydroxypropylacrylat, Hydroxybutylacrylat, Hydroxyethylmethacrylat, Hydroxypropylmethacrylat, Hydroxybutylmethacrylat, Hydroxyhexylmethacrylat und Hydroxyoctylmethacrylat.

Weitere Vinyl-copolymerisierbare Verbindungen können verwendet werden, um einen Teil der verwendbaren Acrylpolymere zu bilden, wie Styrol, Vinyltoluol, Acrylamid, Vinylxylol, Allylalkohol und Acrylnitril.

Bei einem besonders geeigneten Acrylpolymer besteht das Polymer im wesentlichen aus einem harten Bestandteil, nämlich entweder Styrol oder einem Niederalkylmethacrylat, wobei die Acrylgruppe 1 bis 2 Kohlenstoffatome enthält, oder einem Gemisch aus Styrol und Niederalkylmethacrylat, wie Ethylacrylat, einem weichen Bestandteil, nämlich einem Niederalkylmethacrylat mit 3 bis 8 Kohlenstoffatomen in der Alkylgruppe oder Niederalkylacrylat mit 2 bis 8 Kohlenstoffatomen in der Alkylgruppe, einem Hydroxyniederalkylmethacrylat oder -acrylat mit 1 bis 4 Kohlenstoffatomen in der Alkylgruppe und einer α,β-ethylenisch ungesättigten Carbonsäure, wie vorstehend beschrieben.

Neben wasserlöslichen Acrylharzen sind Polyester, die aus gesättigter oder aromatischer Polycarbonsäure und einem Polyol hergestellt sind, für die niederes Molekulargewicht aufweisenden Polymere geeignet. Typische gesättigte aliphatische Dicarbonsäuren sind Anhydride mit 2 bis 10 Kohlenstoffatomen, wie Bernsteinsäure, Azelainsäure und Adipinsäure, die zur Herstellung dieser Polyester geeignet sind. Beispiele von aromatischen zweibasigen Säuren oder deren Anhydride sind Phthalsäure und Trimellitsäure. Die Säuremenge für den Polyester wird so eingestellt, daß die gewünschte Säurezahl erzielt wird, die für den Polyester 20 bis 85 sein sollte. Viele Polyole können mit den vorstehend genannten Säuren zur Herstellung der gewünschten Ester umgesetzt werden. Besonders geeignete Diole sind beispielsweise Ethylenglycol, 1,4-Butandiol, Neopentylglycol, Sorbit, Pentaerythrit und Trimethylolpropan.

Alkydharze, wie Polymerester, hergestellt durch Kondensation von mehrwertigem Alkohol, wie Glycerinethylenglycol, und einer trocknenden Ölfettsäure, wie Leinsamenöl und Tallöl, sind ebenfalls als wasserlösliche Polymere geeignet. Ein weiterer Bestandteil, der gewöhnlich zugegeben wird, um die gewünschte Säurezahl zu erzielen, ist beispielsweise α,β-ethylenisch ungesättigte Dicarbonsäure oder das Anhydrid der Säure, wie Maleinsäure oder Maleinsäureanhydrid.

Vorzugsweise sollten diese Alkydharze ein zahlenmittleres Molekulargewicht von 1 000 bis 2 500 und eine Säurezahl von 20 bis 85 aufweisen.

Ein weiteres niedermolekulares Carbonsäurepolymer, das zur Erzeugung von Elektrodepositionen eingesetzt werden kann, ist ein Polymer aus Styrol und einem 3 bis 10 Kohlenstoffatome enthaltenden, ethylenisch ungesättigten Alkohol, wie Allylalkohol. Das Polymer kann weiterhin mit trocknenden Ölfettsäuren umgesetzt werden und mit einem Säurebestandteil, wie jenen, die vorstehend genannt wurden, um die erforderliche Säurezahl, gewöhnlich im Bereich von 20 bis 80, zu erzielen. Die zahlenmittleren Molekulargewichte der Styrol-Allyl-Alkoholpolymere liegen gewöhnlich im Bereich von 1 000 bis 10 000.

Epoxidester sind ebenfalls als wasserlösliche Harze niederen Molekulargewichts geeignet. Diese Materialien werden durch Teilveresterung eines Epoxidharzes mit einer üblichen trocknenden Ölfettsäure, wie jene, die vorstehend genannt wurden, erzielt und anschließend wird dieses Harz mit einer α,β-ethylenisch ungesättigten Dicarbonsäure oder einem Anhydrid davon, wie jene, die vorstehend erwähnt wurden, verestert. Das Epoxidharz selbst ist vorzugsweise ein Polyglycidylether eines Bisphenols, wie Bisphenol A.

Beispiele weiterer geeigneter harzartiger Polymere niederen Molekulargewichts sind die Neutralisationsprodukte von ungesättigten Carbonsäuren, wie Maleinsäure oder -anhydrid, und einem trocknenden Öl, wie Leinsamenöl.

Eine weitere Zusammensetzung zur anodischen Elektrodeposition umfaßt (A) 15 bis 60 Gewichtsprozent Wasser, (B) 15 bis 60 Gewichtsprozent eines oder mehrerer organischer Lösungsmittel, (C) 0,1 bis 20 Gewichtsprozent eines Copolymers von (a), 10 bis 75 Gewichtsprozent eines oder mehrerer mit Wasser nicht mischbarer oder teilweise mit Wasser mischbarer, copolymerisierbarer α,β-olefinisch ungesättigter Verbindungen, und (b) 25 bis 90 Gewichtsprozent einer oder mehrerer wasserlöslicher copolymerisierbarer N-Vinylverbindungen und (D) 10 bis 79 Gewichtsprozent eines oder mehrerer fein verteilter Pigmente oder Füllstoffe oder Gemische von Pigmenten und Füllstoffen, dispergiert in Gemisch (A), (B), (C), wobei die Summe der Prozentsätze (A), (B), (C), (D) 100 sind. Insbesondere ist hierin Komponente (C) ein Copolymer, das beispielsweise durch Lösungspolymerisation hergestellt werden kann. Es wird in einer Menge von 0,1 bis 20 Gewichtsprozent, vorzugsweise 3 bis 8 Gewichtsprozent, eingesetzt und enthält als copolymerisierbare Einheiten (a) 10 bis 75 Gewichtsprozent, vorzugsweise 20 bis 40 Gewichtsprozent, eines oder mehrerer mit Wasser nicht mischbarer oder nur teilweise mischbarer, copolymerisierbarer α,β-ethylenisch ungesättigter Verbindungen und (b) 25 bis 90 Gewichtsprozent, vorzugsweise 60 bis 80 Gewichtsprozent, eines oder mehrerer wasserlöslicher copolymerisierbarer N-Vinylverbindungen.

Die Summe der Prozentsätze von (a) und (b) ist 100. Bevorzugte, mit Wasser nicht mischbare oder nur teilweise mit Wasser mischbare, α,β-ethylenisch ungesättigte Verbindungen (a) sind Vinylester von C₂-C₁₈-Monocarbonsäuren, beispielsweise Vinylacetat, Vinylpropionat, Vinylpivalat, Vinyl-2-ethylhexanoat und Vinylstearat und/oder Acrylsäureester oder Methacrylsäureester von C₄-C₁₈-Alkoholen, beispielsweise Butylacrylat und Butylmethacrylat, 2-Ethylhexylacrylat, Octylacrylat und Octadecylacrylat. Ein besonders bevorzugtes Monomer ist Vinylpropionat. Geeignet sind auch wasserunlösliche oder nur wenig wasserlösliche Derivate von Acrylamid oder Methacrylamid, Vinylether und/oder Vinylaromaten, wie Styrol.

Beispiele bevorzugter wasserlöslicher N-Vinylverbindungen (b) sind N-Vinylpyrrolidon, N-Vinylpiperidon und N-Vinylimidazol.

Zur kathodischen Elektrobeschichtung von Metallgegenständen geeignete Massen werden nachstehend angeführt. Beispielsweise basiert eine emulgierte Masse auf einem Copolymer, das tertiäre Aminogruppen und blockierte Isocyanatgruppen enthält und das dadurch wasserlöslich oder in Wasser dispergierbar ist, indem es mindestens teilweise mit einer Säure ein Salz bildet, wobei das Copolymer als copolymerisierte Einheiten (A₁) 6 bis 22 Gewichtsprozent eines oder mehrerer Monomere von tertiärer Aminomethacrylsäure oder -acrylsäureester oder -acrylamiden oder -methacrylamiden mit einer tertiären Aminogruppe enthält, (B₁) 21 bis 40 Gewichtsprozent eines monomeren Adduktes eines N-(1)-Alkenylcyanats, wobei 1-Alkenyl 2 bis 4 Kohlenstoffatome aufweist, und eines CH-, OH- oder NH-sauren Blockierungsmittels, (C₁) 0 bis 35 Gewichtsprozent eines oder mehrerer copolymerisierbarer olefinisch ungesättigter Verbindungen, die aktive Wasserstoffatome aufweisen, und die mit Isocyanatgruppen reaktiv sind, und (D₁) 3 bis 73 Gewichtsprozent olefinisch ungesättigte Verbindungen, die nicht unter (A₁) bis (C₁) genannt wurden, ausgewählt aus der Gruppe, bestehend aus Estern von Acrylsäure und Methacrylsäure mit Monoalkoholen mit 1 bis 18 Kohlenstoffatomen, Vinylester von Carbonsäuren mit 2 bis 10 Kohlenstoffatomen, Vinylaromaten, Acrylnitril von ungesättigten Triglyceriden, wobei das Copolymer ein mittleres Molekulargewicht von 1 000 bis 20 000 aufweist, und die Prozentsätze von (A₁) bis (D₁) insgesamt 100 sind. Geeignete Komponenten (A) sind ethylenisch ungesättigte Verbindungen, die tertiäre Aminogruppen aufweisen, wie tertiäre Aminomethacryl- oder Acrylsäureester, beispielsweise Dialkylaminoalkylacrylate und -methacrylate, wobei Alkyl 1 bis 8 Kohlenstoffatome aufweist, beispielsweise N,N-Dimethylaminoethylmethacrylat und N,N-Diethylaminoethylacrylat oder -acrylamide oder -methacrylamide, die eine tertiäre Aminogruppe enthalten, beispielsweise N,N-Dimethylaminopropylacrylamid oder -methacrylamid und N,N-Diethylaminopropylacrylamid oder -methacrylamid.

Das Bindemittel enthält 6 bis 22, vorzugsweise 6 bis 15 Gewichtsprozent Komponente (A₁) als copolymerisierte Einheit. Die Verwendung von 6 bis 10 Gewichtsprozent der vorstehend genannten Acrylamide, die eine Aminogruppe enthalten, ist besonders bevorzugt.

Komponente (B₁) ist ein Addukt von N-(1-Alkenyl)isocyanat und eines CH-, OH- oder NH-sauren Blockierungsmittels. Geeignete N-(1-Alkenyl)isocyanate sind jene, worin Alkenyl 2 bis 4 Kohlenstoffatome aufweist, vorzugsweise Vinylisocyanat und/oder Propenylisocyanat. Beispiele geeigneter Blockierungsmittel zur Herstellung von Komponente (B₁) sind Monophenole, beispielsweise Phenol, Cresol und Trimethylphenol, primäre Alkohole und sekundäre Alkohole, beispielsweise Isopropanol und Cyclohexanol, tertiäre Alkohole, beispielsweise tert-Butanol und tert-Amylalkohol, leicht enolisierbare Verbindungen, beispielsweise Ethylacetoacetat, Acetylaceton, Malonsäurederivate, beispielsweise Malonsäurediester mit Alkoholen von 1 bis 8 Kohlenstoffatomen, Malonitril, sekundäre aromatische Amine, beispielsweise N-Methylanilin, N-Methyltoluidin und N-Phenyltoluidin, Imide, beispielsweise Succinimid und Phthalimid, Lactam, beispielsweise ε-Caprolactam, δ-Valerolactam und Lauryllactam, sowie Oxime, beispielsweise Acetonoxim, Butanonoxim und Cyclohexanonoxim. Besonders bevorzugte Blockierungsmittel für N-(1-Alkenyl)isocyanate sind tert-Butanol, Cyclohexanol und Caprolactam.

Die Herstellung von blockiertem N-(Alk-1-enyl)isocyanat, beispielsweise Vinylisocyanat, kann beispielsweise durch Verfahren in Gegenwart eines Lösungsmittels hergestellt werden. Etwa gleichmolare Mengen werden zur Umsetzung von N-(Alk-1-enyl)isocyanat(phenylisocyanat) als Blockierungsmittel verwendet. Ein Überschuß von Isocyanat sollte vermieden werden, da dies schließlich zu Vernetzung führen könnte. Komponente (B) liegt in dem Copolymer in Mengen von 20 bis 40, vorzugsweise 25 bis 33, Gewichtsprozent in Form von copolymerisierten Einheiten vor.

Geeignete reaktive Monomere (C₁) zum Copolymerisieren sind olefinisch ungesättigte Verbindungen, die aktive Wasserstoffatome enthalten, welche für Isocyanatgruppen reaktiv sind, das heißt welche beispielsweise OH- und NH-Gruppen tragen. Beispiele dafür sind Monoester von Acrylsäure oder Acrylsäure mit mehrwertigen, insbesondere zweiwertigen Alkoholen, beispielsweise Hydroxyethylacrylat und -methacrylat, Ethoxypropylacrylat und -methacrylat und Hydroxybutylacrylat und Monoester von diesen Säuren mit Polyetherdiolen, beispielsweise Polypropylenglycolacrylat und -methacrylat sowie Allylalkoholbut-1-en-3,4-diol. N-Methylolacrylamid und N-Ethylolacrylamid können ebenfalls verwendet werden. Ester von Acrylsäure oder Methacrylsäure mit Diolen von 2 bis 4 Kohlenstoffatomen, insbesondere Hydroxypropylacrylat und Hydroxyethylacrylat, sind besonders bevorzugt.

Komponente (C₁) liegt in dem Copolymer in einer Menge von 0 bis 35, vorzugsweise 20 bis 30, Gewichtsprozent als copolymerisierte Einheiten vor.

Geeignete Komponenten (D₁) sind copolymerisierbare olefinisch ungesättigte Verbindungen, die nicht unter (A₁) bis (C₁) genannt wurden, beispielsweise Ester von Acrylsäure und Methacrylsäure mit Monoalkoholen mit 1 bis 18, vorzugsweise 1 bis 8 Kohlenstoffatomen, beispielsweise Methylacrylat, Ethylacrylat, Butylacrylat, Ethylhexylacrylat und Methylmethacrylat. Alle weiteren copolymerisierbaren ungesättigten Verbindungen können ebenfalls verwendet werden, insbesondere Vinylester von Carbonsäuren mit 2 bis 10 Kohlenstoffatomen, wie Vinylacetat, Vinylaromaten, beispielsweise Styrol, Acrylnitril und ungesättigte Triglyceride, beispielsweise isomerisiertes Leinsamenöl. Die Copolymere werden vorzugsweise in polaren Lösungsmitteln ohne OH-Gruppen hergestellt, beispielsweise Ether, z.B. Tetrahydrofuran, oder Ester, z.B. Ethylacetat oder N-Butylacetat, in Gegenwart von Radikalstartern, wie Azobiscarboxamiden, Azobiscarbonsäurenitrilen und Peroxiden, im allgemeinen bei 50 bis 120°C, vorzugsweise 60 bis 90°C, in Gegenwart oder Abwesenheit von Regulierungsmitteln, beispielsweise tert-Decylmercaptan und Diisopropylxanthogendisulfid.

Eine weitere kationische Beschichtungsmasse umfaßt ein wässeriges Medium und ein darin dispergiertes harzartiges Bindemittel, wobei das harzartige Bindemittel durch Inkontaktbringen mindestens eines Ausgangsmaterials mit einer teilweise blockierten Polyisocyanatverbindung bei einer Temperatur von 40° bis 130°C in einem Gewichtsverhältnis von 5 bis 9 des Ersteren mit 5 bis 1 des Letzteren und anschließend Neutralisieren des erhaltenen Produkts mit einer Säure hergestellt wird, wobei das Ausgangsmaterial mindestens eines ist, ausgewählt aus der Gruppe, bestehend aus
1. einem Gemisch eines Reaktionsproduktes, hergestellt durch Umsetzung eines Epoxidharzes mit einer basischen Aminoverbindung mit mindestens einer basischen Aminogruppe und einem Polyamid mit mindestens einer basischen Aminogruppe in einem Gewichtsverhältnis von 1 bis 9 des Reaktionsprodukts zu 9 bis 1 des Polyamids und
2. einem weiteren Reaktionsprodukt, hergestellt durch Vermischen des vorstehend genannten Reaktionsproduktes mit dem Polyamid bei einer Temperatur von 50° bis 200°C, in einem Gewichtsverhältnis von 1 bis 9 des Reaktionsproduktes zu 9 bis 1 des Polyamids, wobei die teilweise blockierte Polyisocyanatverbindung mit mindestens einer blockierten Isocyanatgruppe in dem Molekül vorliegt und im Durchschnitt mehr als 0 bis nicht mehr als 1 freie Isocyanatgruppe pro Molekül aufweist.

Die Komponente 1. ist ein Reaktionsprodukt, erhalten durch Umsetzung eines Epoxidharzes mit einer basischen Aminoverbindung. Verwendbare Epoxidharze sind jene, die aus einer phenolischen Verbindung und Epichlorhydrin, saurem Epoxidharz, das mindestens zwei Epoxygruppen pro Molekül enthält, erhalten werden und weisen gewöhnlich ein Molekulargewicht von etwa 200 bis 4 000, vorzugsweise etwa 400 bis 2 000, auf. Besondere Beispiele der Harze vom Vinyltyp sind ein Epoxidharz, hergestellt aus Bisphenol A und Epichlorhydrin, ein Epoxidharz, hergestellt aus hydriertem Bisphenol A und Epichlorhydrin, ein Epoxidharz, hergestellt aus Bisphenol A und β-Methylenepichlorhydrin, Polyglycidylether von Novolakharz, usw., wobei ein Epoxidharz, erhalten aus Bisphenol A und Epichlorhydrin, besonders bevorzugt ist. Ein solches Epoxidharz vom Phenoltyp kann gemeinsam mit einer Polyepoxyverbindung, wie Polyglycidylether von Ethylenglycol, Propylenglycol, Glycerin, Trimethylolpropan, und dergleichen, mehrwertigem Alkohol, Polyglycidylester von Adipinsäure, Phthalsäure, dimerer Säure oder dergleichen, Polycarbonsäure, Polyepoxiden, erhalten durch Epoxidieren von alicyclischern Olefin und 1,2-Polybutadien, usw., verwendet werden. Die Einsatzmenge kann bis zu etwa 25 Gewichtsprozent betragen.

Beispielhafte basische Aminosäuren, die mit dem Epoxidharz umzusetzen sind, sind aliphatische oder alicyclische Aminoverbindungen mit einer primären oder sekundären Aminogruppe. Bevorzugte Beispiele sind Monoamine, wie Mono- oder Dialkylamin, Mono- oder Dialkanolamine und Polyamine, wie Polyalkylenpolyamine, usw.. Geeignete Monoamine sind Mono- oder Dialkylamine mit etwa 1 bis 18 Kohlenstoffatomen, wie Propylamin, Butylamin, Diethylamin, Dipropylamin, usw.. Beispiele von Mono- oder Dialkanolmonoaminen sind Ethanolamin, Propanolamin, Diethanolamin, Dipropanolamin, usw.. Geeignete Beispiele von anderen Monoaminen sind Piperidin, Cyclohexylamin, Pyrrolidin, Morpholin, usw.. Beispiele von Polyaminen sind Ethylendiamin, Hexamethylendiamin, Triethylentriamin, Triethylentetramin, Tetraethylenpentamin, Propylendiamin, Dipropylentriamin, Butylendiamin, N-Aminoethanolamin, Monoethylethylendiamin, Diethylaminopropylamin, Hydroxyethylaminopropylamin, Monomethylaminopropylamin, Piperazin, N-Methylpiperazin, N-Aminoethylpiperazin, usw.. Besonders geeignet sind aliphatische Mono- oder Polyamine mit einer sekundären Aminogruppe, wie Diethylamin, Diethanolamin, Diethylentriamin, Monoethylethylendiamin, Hydroxyethylaminopropylamin, usw., hinsichtlich der Reaktivität mit Epoxidharz. Ein aromatisches Amin kann in Kombination mit einem aliphatischen oder alicyclischen Amin in einer Menge verwendet werden, daß das Reaktionsprodukt von Epoxidharz und basischem Amin, neutralisiert mit einer Säure, noch in Wasser dispergiert verbleibt. Beispiele geeigneter aromatischer Amine sind Anilin, N-Methylanilin, Toluidin, Benzylamin, m-Xylylendiamin, m-Phenylendiamin, 4,4-Diaminodiphenylmethan, usw..

Die Umsetzung von Epoxidharz mit basischen Aminoverbindungen erfolgt in an sich bekannter Weise. -

Beispiele von Polyamidharz mit einer basischen Aminogruppe, das einzumischen oder mit Komponente (1) umzusetzen ist, sind jene, die in dem Molekül mindestens eine Aminogruppe oder mindestens eine Amidogruppe enthalten, die mit der Isocyanatgruppe der teilweise blockierten Polyisocyanatverbindung reagieren kann. Insbesondere sind Beispiele Polyamide, hergestellt durch Kondensation einer Dicarbonsäure und Polyamin, durch Umsetzung eines Polyamins mit einem Oligomer, hergestellt durch Ringöffnungspolymerisation von Lactam, wie ε-Caprolactam oder Polyesterpolyamid von Alkanolamin und Dicarbonsäuren, usw. bevorzugt. Die Dicarbonsäuren sind jene, wiedergegeben durch die allgemeine Formel

HOOC-R-COOH

wobei R eine gesättigte oder ungesättigte aliphatische Kohlenwasserstoffgruppe oder eine aromatische Kohlenwasserstoffgruppe mit 1 bis 34 Kohlenstoffatomen ist. Bevorzugte Beispiele sind Phthalsäure, Malonsäure, Maleinsäure, Fumarsäure, Bernsteinsäure, Azelainsäure, Adipinsäure, Sebacinsäure, Dodecylbernsteinsäure, dimere Säure, usw.. Die Polyamine sind Polyalkylenpolyamine mit primärer Aminogruppe an beiden Enden der Hauptkette, wiedergegeben durch die allgemeinen Formeln 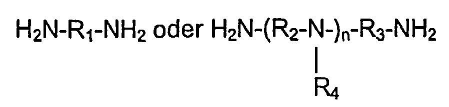 worin R₁, R₂ und R₃ aliphatische Kohlenwasserstoffgruppen mit 2 bis 6 Kohlenstoffatomen sind, R₄ Wasserstoff oder eine aliphatische Kohlenwasserstoffgruppe mit 1 bis 3 Kohlenstoffatomen ist und n eine ganze Zahl von 1 bis 6 ist. Bevorzugte Beispiele sind Ethylendiamin, Propylendiamin, Butylendiamin, Hexamethylendiamin, Tetraethylenpentamin, Pentaethylenhexamin, Hexamethylenheptamin, Hexaethylenoctamin, Diethylentriamin, Triethylentetramin, Bis(3-aminopropyl)amin, 1,3-Bis(3-aminopropylamino)propan, usw.. Geeignete Alkanolamine schließen jene mit 2 bis 6 Kohlenstoffatomen, wie Ethanolamin, Propanolamin, Hydroxyethylaminopropylamin, usw. ein.

Ein geeignetes Verfahren zur Herstellung eines kathodisch abscheidbaren Elektrodepositionsfilms, umfaßt die Umsetzung von
(A₂) einer ungesättigten organischen Verbindung mit einem Molekulargewicht von 300 bis 30 000 mit einer Kohlenstoff-Kohlenstoff-Doppelbindung in einer Menge, entsprechend einer Jodzahl von 50 bis 500, wobei die ungesättigte organische Verbindung ausgewählt ist aus der Gruppe, bestehend aus (a) einem Polymer eines konjugierten Diolefins mit 4 bis 8 Kohlenstoffatomen, (b) einem Copolymer von mindestens zwei konjugierten Diolefinen, enthaltend 4 bis 8 Kohlenstoffatomen, (c) einem Copolymer von mindestens einem konjugierten Diolefin, enthaltend 4 bis 8 Kohlenstoffatome und einem Vinylmonomer mit einer ethylenischen Ungesättigtheit mit 2 bis 20 Kohlenstoffatomen, (d) einem natürlichen Öl, (e) einem natürlichen Fett und (f) einem Mineralölharz, erzeugt durch kationische Polymerisation von Mineralöl-Crackfraktionen mit 4 bis 10 Kohlenstoffatomen mit einem Friedel-Crafts-Katalysator, wobei die ungesättigte organische Verbindung über Kohlenstoff-Kohlenstoff-Bindung daran gebundene Epoxidgruppen aufweist der Formel 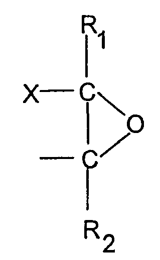
worin R₁ und R₂ unabhängig voneinander Wasserstoff oder Methyl darstellen und X ein Wasserstoffatom oder eine Bindung darstellt, und wenn X eine Bindung darstellt, das Kohlenstoffatom, an das R₁ gebunden ist, und das Kohlenstoffatom, an das R₂ gebunden ist, Teil einer Hauptkette der Komponente (A) bilden können,
wobei die Menge an Epoxygruppen in der Komponente (A₂) 0,05 bis 0,2 Mol pro 100 g der Komponente (A₂) beträgt,
mit (B₂) einer primären oder sekundären Aminverbindung der Formel 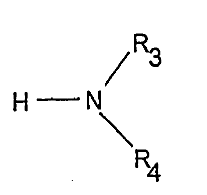 worin R₃ und R₄ gleich oder verschieden Kohlenwasserstoffgruppen mit 1 bis 10 Kohlenstoffatomen darstellen, wobei jedes von R₃ und R₄ ein Wasserstoffatom sein kann,
bei einer Temperatur von 100° bis 200°C zur Herstellung eines harzartigen Stoffes, der basische Gruppen und Hydroxidgruppen enthält, durch Zugabe einer wasserlöslichen anorganischen oder organischen Säure zu dem harzartigen Stoff, um den harzartigen Stoff wasserlöslich zu gestalten, und Vermischen des erhaltenen wasserlöslichen, harzartigen Stoffes mit einem wässerigen oder organischen flüssigen Medium, oder Ausführen der vorstehenden Umsetzung in Gegenwart eines flüssigen Mediums.

Beispiele einer solchen ungesättigten organischen Verbindung sind natürliche Öle und Fette, wie Leinsamenöl, Tungöl, Sojabohnenöl oder dehydriertes Rizinusöl und Öle, hergestellt durch Wärmebehandlung von Naturölen und Fetten zur Erhöhung ihres Molekulargewichts. Beispiele für eine Flüssigkeit, die ungesättigte Gruppen enthält oder eines festen Polymers sind Polymere mit geringem Polymerisationsgrad von konjugierten Diolefinen, gewöhnlich mit 4 bis 8 Kohlenstoffatomen, wie Butadien, Isopren oder Piperylen, Copolymere mit geringem Polymerisationsgrad von zwei oder mehr dieser konjugierten Diene oder Copolymere mit geringem Polymerisationsgrad von mindestens einem dieser konjugierten Olefine, und ein Vinylmonomer mit einer ethylenischen Ungesättigtheit, gewöhnlich mit 2 bis 20 Kohlenstoffatomen, insbesondere aliphatische oder aromatische Vinylmonomere, wie Isobutylen, Diisobutylen, Acryl- oder Methacrylsäure oder Ester davon, Allylalkohol oder dessen Ester, Styrol, α-Methylstyrol, Vinyltoluol oder Divinylbenzol. Diese Verbindungen können einzeln oder in Anmischung von zwei oder mehreren verwendet werden.

Außerdem ist ein geeignetes Verfahren ein Verfahren zur Elektrobeschichtung einer elektrisch leitfähigen Oberfläche, die als Kathode dient, umfassend Leiten von elektrischem Strom zwischen Kathode und Anode in Kontakt mit einer wässerigen Elektrodepositionszusammensetzung,
wobei die Elektrodepositionszusammensetzung umfaßt:
ein Säure-lösliches, selbsthärtendes synthetisches organisches Harz mit Aminogruppen, Hydroxylgruppen und blockierten Isocyanatgruppen, wobei die blockierten Isocyanatgruppen bei Raumtemperatur in Gegenwart von Hydroxyl- oder Aminogruppen stabil sind und mit Hydroxylgruppen oder Aminogruppen bei erhöhten Temperaturen reaktiv sind und wobei das organische Harz von dem Reaktionsprodukt von
   1. einer Epoxygruppen-enthaltenden organischen Verbindung,
   2. einem primären oder sekundären Amin und
   3. einem teilweise blockierten organischen Polyisocyanat, abgeleitet ist.

Das Epoxidmaterial, das verwendet wird, kann ein beliebiges monomeres oder polymeres Material mit durchschnittlich einer oder mehren Epoxygruppen pro Molekül sein. Die Monoepoxide können verwendet werden, vorzugsweise ist die Epoxidverbindung harzartig und vorzugsweise ein Polyepoxid mit zwei oder mehreren Epoxygruppen pro Molekül. Das Epoxidharz kann im wesentlichen ein beliebiges gut bekanntes Epoxid sein. Eine besonders brauchbare Klasse von Polyepoxiden sind die Polyglycidylether von Polyphenolen, wie Bisphenol A. Diese können beispielsweise durch Verethern eines Polyphenols mit Epichlorhydrin in Gegenwart eines Alkali hergestellt werden. Die Phenolverbindung kann beispielsweise Bis(4-hydroxyphenyl)-2,2-propan, 4,4'-Dihydroxybenzophenon, Bis(4-hydroxyphenyl)-1,1-ethan, Bis(4-hydroxyphenyl)-1,1-isobutan, Bis(4-hydroxy-tertiär-butylphenyl)-2,2-propan, Bis(2-hydroxynaphthyl)methan, 1,5-Dihydroxynaphthylen oder dergleichen sein. In vielen Fällen ist es erwünscht, Polyepoxide mit etwas höherem Molekulargewicht, die aromatische Gruppen enthalten, zu verwenden.

Geeignete ähnliche Polyglycidylether von mehrwertigen Alkoholen können von mehrwertigen Alkoholen, wie Ethylenglycol, Diethylenglycol, Triethylenglycol, 1,2-Propylenglycol, 1,4-Propylenglycol, 1,5-Pentandiol, 1,2,6-Hexantriol, Glycerin, Bis(4-hydroxycyclohexyl)2,2-propan und dergleichen, abgeleitet sein. Es können auch Polyglycidylester von Polycarbonsäuren, die beispielsweise durch Umsetzung von Epichlorhydrin oder ähnlichen Epoxidverbindungen mit einer aliphatischen oder aromatischen Polycarbonsäure, wie Oxalsäure, Bernsteinsäure, Glutarsäure, Terephthalsäure, 2,6-Naphthylandicarbonsäure, dimerisierter Linolsäure und dergleichen, hergestellt werden, eingesetzt werden.

Beispiele sind Glycidyladipat und Glycidylphthalat. Ebenfalls geeignet sind Polyepoxide, die von der Epoxidierung von olefinisch ungesättigten alicyclischen Verbindungen abgeleitet sind. Eingeschlossen sind die zum Teil eines oder mehrere Monoepoxide umfassende Epoxide. Diese Polyepoxide sind nicht phenolisch und werden durch Epoxidieren von alicyclischen Olefinen, beispielsweise mit Sauerstoff und ausgewählten Verfahrenskatalysatoren, beispielsweise Perbenzoesäure, durch Acetaldehydmonoperacetat oder durch Persäure hergestellt. Unter solchen Polyepoxiden sind die Epoxy-alicyclischen Ether und Ester bekannt. Weitere Epoxid-enthaltende Verbindungen und Harze, die Stickstoffenthaltende Diepoxide enthalten, sind in US-A-3 365 471 beschrieben, wie Epoxidharze mit 1,1-Methylenbis(5-substituiertem)hydantoin, (US-A-3 391 097), Bisimid-enthaltende Diepoxide (US-A-3 450 711), epoxydierte Aminmethyldiphenyloxide (US-A-3 312 664), heterocyclische N,N'-Diglycidylverbindungen (US-A-3 503 979), Aminoepoxyphosphonate (GB-A-1 172 916), 1,3,5-Triglycidylisocyanurate.

Die teilweise oder halb verkappten oder blockierten Isocyanate, die bei der Herstellung der verwendeten Verbindungen eingesetzt werden können, können beliebige Polyisocyanate sein, bei denen der Anteil der Isocyanatgruppen mit einer Verbindung so umgesetzt wurde, daß der erhaltene verkappte Isocyanatteil gegen Hydroxyl- oder Amingruppen bei Raumtemperatur stabil ist, allerdings bei höheren Temperaturen gewöhnlich oberhalb 90°C und etwa 315°C mit Hydroxyl- oder Aminogruppen reagiert. Die halb verkappten Polyisocyanate sollten im Durchschnitt etwa eine freie reaktive Gruppe enthalten.

Bei der Herstellung von teilweise blockierten organischen Polyisocyanaten können beliebige geeignete organische Polyisocyanate verwendet werden. Beispiele sind aliphatische Verbindungen, wie Trimethylen-, Tetramethylen-, Pentamethylen-, Hexamethylen-, 1,2-Propylen-, 1,2-Butylen-, 2,3-Butylen-, 1,3-Butylen-, Ethyliden- und Butylidendiisocyanate, die Cycloalkylenverbindungen, wie 1,3-Cyclopentan-, 1,4-Cyclohexan- und 1,2-Cyclohexandiisocyanate, die aromatischen Verbindungen, wie m-Phenylen-, p-Phenylen-, 4,4'-Diphenyl-, 1,5-Naphthalin- und 1,4-Naphthalindiisocyanate, die aliphatisch-aromatischen Verbindungen, wie 4,4'-Diphenylenmethan-, 2,4- oder 2,6-Tolylen- oder Gemische davon, 4,4'-Toluidin- und 1,4-Xylylendiisocyanate, die kernsubstituierten aromatischen Verbindungen, wie Dianisidindiisocyanat, 4,4'-Diphenyletherdiisocyanat- und Chlordiphenylendiisocyanat, die -triisocyanate, wie Triphenylmethan-4,4',4"-triisocyanat, 1,3,5-Triisocyanatbenzol und 2,4,6-Triisocyanattoluol und die -tetraisocyanate, wie 4,4'-Diphenyldimethylmethan-2,2',5,5'-tetraisocyanat, die polymerisierten Polyisocyanate, wie Tolylendiisocyanatdimere und -trimere und dergleichen.

Vorzugsweise sollten die angewendeten Polyisocyanate Isocyanatgruppen mit unterschiedlicher Reaktivität aufweisen, um die teilweise Blockierungsreaktion zu erleichtern.

Außerdem können organische Polyisocyanate ein Prepolymer darstellen, abgeleitet von einem Polyol, einschließlich Polyetherpolyol oder Polyesterpolyol.

Beliebige geeignete aliphatische, cycloaliphatische aromatische Alkylmonoalkohol- und Phenolverbindungen, die als Blockierungsmittel verwendet werden können, sind beispielsweise niederaliphatische Alkohole, wie Methyl-, Ethyl-, Chlorethyl-, Propyl-, Butyl-, Amyl-, Hexyl-, Heptyl-, Octyl-, Nonyl-, 3,3,5-Trimethylhexanol-, Decyl- und Laurylalkohole und dergleichen, und cycloaliphatische Alkohole, wie beispielsweise Cyclopentanol, Cyclohexanol und dergleichen; die aromatischen Alkylalkohole, wie Phenylcarbinol, Methylphenylcarbinol, Ethylenglycolmonoethylether, Ethylenglycolmonobutylether und dergleichen; die Phenolverbindungen, wie Phenol selbst, einschließlich Phenole, die substituiert sind. Beispiele sind Cresol, Xylenol, Nitrophenol, Chlorphenol, Ethylphenol, t-Butylphenol und 2,5-Di-t-butyl-4-hydroxytoluol.

Weitere Blockierungsmittel schließen tertiäre Hydroxylamine, wie Diethylethanolamin und Oxime, wie Methylethylketonoxim, Acetonoxim und Cyclohexanonoxim, ein. Die Verwendung von Oximen und Phenolen ist besonders erwünscht, da Polyisocyanate, die mit diesen Mitteln blockiert sind, bei relativ geringen Temperaturen ohne äußere Zugabe von Urethan-bildenden Katalysatoren, wie Zinnkatalysatoren, deblockieren.

Halbverkappte organische Polyisocyanate werden durch Umsetzen einer ausreichenden Menge von Blockierungsmittel mit organischem Polyisocyanat hergestellt, unter Bereitstellung eines Produktes mit freien Isocyanatgruppen, die verbleiben.

Das Epoxid-enthaltende Material wird mit einem Amin unter Herstellung eines Addukts umgesetzt. Das angewendete Amin kann ein beliebiges primäres oder sekundäres Amin, vorzugsweise sekundäres Amin, sein. Vorzugsweise ist das Amin eine wasserlösliche Aminverbindung. Beispiele solcher Amine schließen Mono- und Dialkylamine, wie Methylamin, Ethylamin, Propylamin, Butylamin, Dimethylamin, Diethylamin, Dipropylamin, Dibutylamin, Methylbutylamin und dergleichen, ein.

Beispiele geeigneter Polymere niederen Molekulargewichts für kathodische Lacke, die Acrylcopolymere, welche vorstehend genannt sind, darstellen, und welche zum Teil eine Monomerladung aufweisen, sind jene, die ein tertiäres Amin enthaltendes Acrylat oder Methacrylat enthalten, wie Dimethylaminoethylmethacrylat, Diethylaminoethylacrylat, Diethylaminoacrylat, und dergleichen. Diese Polymere können in Wasser unter Zusatz von in Wasser dispergierbarer Säure, wie Essigsäure, aufgelöst und dispergiert werden oder können mit einem Alkylierungsmittel, wie Methyljodid oder Dimethylsulfat, quaternisiert werden, um die erforderliche kationische Ladung zu erzielen. Außerdem können auch eine tertiäre Aminogruppe enthaltende Acrylate und Methacrylate, Monomere, wie Methylvinylpyridin und dergleichen, eingesetzt werden.

Weitere Beispiele für Polymermaterialien niederen Molekulargewichts, die eine anionische Ladung enthalten, sind ein Reaktionsprodukt von Polyepoxiden, wie Polyglycidylethern und von Polyphenolen, die vorstehend genannt wurden, umgesetzt mit sekundärem Amin, wie Dimethylaminodiethylamin. Diese Addukte können dann mit Säuren neutralisiert werden oder wie vorstehend beschrieben quaternisiert werden, unter Bereitstellung der erforderlichen kationischen Gruppen.

Alle vorstehend genannten Harze können auch wasserunlösliche harzartige Materialien enthalten. Diese Materialien sind polymer und werden im wesentlichen aus hydrophoben polymerisierbaren Reaktanten hergestellt, wie ethylenisch ungesättigten Monomerzusammensetzungen, die ein oder mehrere polymerisierbare, ethylenisch ungesättigte Verbindungen enthalten, welche jeweils miteinander polymerisiert, wasserunlösliche Polymere bilden. Die polymerisierbaren, ethylenisch ungesättigten Verbindungen werden wiedergegeben durch nichtionische Monomere, wie die Alkenyl-aromatischen Verbindungen, das heißt die Styrolverbindungen, die Derivate von α,β-ungesättigten Monocarbonsäuren, wie Acrylester, Acrylnitrile und Methacrylsäureester, Derivate von α,β-ethylenisch ungesättigten Dicarbonsäuren, wie Maleinsäureestern, ungesättigte Alkoholester, konjugierte Diene, ungesättigte Ketone, ungesättigte Ether und andere polymerisierbare Vinylidenverbindungen, wie Vinylchlorid und Vinylidenfluorid. Spezielle Beispiele solcher ethylenisch ungesättigter Verbindungen sind Styrol, α-Methylstyrol, α-Ethylstyrol, Dimethylstyrol, Diethylstyrol, t-Butylstyrol, Vinylnaphthalin, Hydroxystyrol, Methoxystyrol, Cyanostyrol, Acetylstyrol, Monochlorstyrol, Dichlorstyrol und andere Halogenstyrole, Methylmethacrylat, Ethylacrylat, Butylacrylat, Hexylacrylat, 2-Ethylhexylacrylat, Laurylmethacrylat, Phenylacrylat, 2-Hydroxybutylacrylat, 2-Hydroxybutylmethacrylat, 4-Hydroxybutylacrylat und 4-Hydroxybutylmethacrylat, Acrylnitril, Methacrylnitril, Acrylanilid, Ethyl-αchloracrylat, Ethylmaleat, Vinylacetat, Vinylpropionat, Vinylchlorid, Vinylbromid, Vinylidenchlorid, Vinylidenfluorid, Vinylmethylketon, Methylisopropenylketon, Vinylethylether, 1,3-Butadien und Isopren.

Bevorzugte Elektrodepositionsemulsionen sind u.a. anodische Elektrodepositionsharzemulsionen, insbesondere der Firma BASF, beispielsweise der Depositionslack ZQ8-43225 von BASF Coatings, Münster.

Als Stoff 4 mit Erkennungsfunktion können beliebige Stoffe, vorzugsweise in Nano- oder Mikroteilchengröße, die mit einer sogenannten Erkennungsfunktion ausgestattet wird, verwendet werden. Die Teilchengrößen betragen 10⁻⁵ bis 10⁻⁸ cm z.B. 10⁻⁶ bis 10⁻⁷ cm, gegebenenfalls 10⁻⁶ bis 10⁻⁵ cm, aber auch 10⁻⁷ bis 10⁻⁵ m, wobei die Bereiche auch das Zehn- oder Hundertfache der Bereichswerte aufweisen können.

Unter dem Begriff "Erkennungsfunktion" wird die Eigenschaft dieser Stoffe verstanden, durch eine ihnen eigene physikalische, biologische, biochemische oder chemische Eigenschaft spezifisch erkannt werden zu können. Die Erkennungsmechanismen können beispielsweise aufgrund von Lichtbrechung, Färbung, Fluoreszenz, Phosphoreszenz, Magnetismus oder Radioaktivität erfolgen, aber auch auf chemischen, insbesondere elektrochemischen Eigenschaften, wie Amperometrie, Impedimetrie oder Voltammetrie, Redoxvorgängen etc., biologischen und/oder insbesondere biochemischen Eigenschaften beruhen. Besonders bevorzugte Stoffe mit Erkennungsfunktion sind Latexbeads, Glasbeads, Graphitteilchen, färbende, fluoreszierende, phosphoreszierende, magnetische und/oder radioaktive Teilchen und/oder Teilchen mit chemischer, biologischer und/oder biochemischer Funktion. Teilchen mit biologischer und/oder biochemischer Funktion sind insbesondere Enzyme, Antikörper, Antigene, Haptene, DNA, RNA, Oligonukleotide, Peptide, Oligopeptide, Proteine, Lectine, Hormone, Rezeptorantagonisten oder -agonisten, Zellen und Mikroorganismen. Insbesondere ist der Stoff mit Erkennungsfunktion ein Enzym.

Als Enzym können beliebige Enzyme, die den Elektrodepositionsvorgang überstehen, bzw. mit der eingesetzten Depositionslackemulsion verträglich sind, verwendet werden. Besonders bevorzugt sind Oxidasen, wie Glucoseoxidase, Peroxidase oder Lactatoxidase. Es ist aber auch denkbar, durch Mehrschichtaufbau coenzymabhängige Enzymverbundschichten aufzubauen, wobei dann insbesondere NADH- bzw. NADPH-abhängige Dehydrogenasen oder PQQabhängige Dehydrogenasen eingesetzt werden können. Des weiteren ist es möglich, durch eine Mehrschichtausbildung auch Oxidasen (z.B. Ascorbatoxidase) und Katalase, letztere als Schutzschicht, zu kombinieren. Außerdem können auch andere Enzyme in mehreren Schichten funktionell angeordnet werden.

Insbesondere läßt sich das erfindungsgemäße Verfahren zur Durchführung von Untersuchungen im Rahmen der chemischen Kombinatorik einsetzen. Zum Beispiel können unterschiedliche, potentiell katalytische Stoffe gezielt über eine punktweise Abscheidung auf einer flächigen Elektrode abgeschieden werden und durch gezielte Abfrage bzw. Abtastung (z.B. elektronisch) der abgeschiedenen Stoffe deren katalytische Beschaffenheit oder sogar ihre Aktivität ermittelt werden.

Zu derartigen Stoffen gehören Peptide, Proteine, aber auch anorganische Stoffe, wie Cu/ZnO, Au/TiO₂ oder Na⁺-dotierte Übergangsmetalle. Denkbar sind auch Katalysatoren auf der Basis klassischer Katalysatoren wie jene der VIII. Nebengruppe des Periodensystems, insbesondere Pt oder Pd.

Da die abgeschiedenen Polymere der Harzschicht 3 ein Gerüst aufweisen, das den Durchgang von Molekülen bestimmter Größe zuläßt, ist es außerdem über die Einstellung der Abscheidungsparameter möglich, Stoffe unterschiedlicher Größe, die gemeinsam in einer Lösung vorliegen, nach ihrer Größe von der abgeschiedenen Schicht auszuschließen bzw. durchzulassen. Es wird ein Filtereffekt erzielt. Dieser kann in vielfältiger Weise - auch zu chemisch kombinatorischen Untersuchungen - eingesetzt werden. Er kann auch bei den vorstehend angeführten Abscheidungen mit Enzymen, insbesondere bei Mehrschichtausbildungen, eingesetzt werden.

Der handelsüblichen Elektrodepositionslacksuspension kann gegebenenfalls Wasser zugesetzt werden, wobei das Wasser möglichst rein sein sollte. Bevorzugt ist Wasser mit HPLC-Qualität. Des weiteren können beliebige andere Stoffe, die Harzschichten üblicherweise zugesetzt werden, eingesetzt werden. Solche Stoffe sind z.B. Weichmacher, Emulgatoren, Adhäsionsverstärker, Hydrophobier- oder Hydrophilierstoffe, Netzmittel, Antischäummittel oder dergleichen.

Geeignete Zusätze, die hauptsächliche die Funktion als Emulgator oder Netzmittel bei der Herstellung und Zubereitung der Elektrodepositionslacksuspension ausüben sind anionische, kationische oder amphophile Tenside, vor allem nichtionogene Tenside. Nachstehend sind diesbezügliche geeignete Handelsprodukte angeführt:
1. Triton (X-100, X-114, X-405 usw.): Alkylphenylpolyethylenglykol (Fluka)
2. Tween (20, 40, 60 usw.):
   - Tween 20: Polyoxyethylensorbitanmonolaurat (Merck)
   - Tween 40: Polyoxyethylensorbitanmonopalmitat (Merck)
   - Tween 60: Polyoxyethylensorbitanmonostearat (Merck)
   - Tween 65: Polyoxyethylensorbitantristearat (Merck)
   - Tween 80: Polyoxyethylensorbitanmonooleat (Merck)
   - Tween 85: Polyoxyethylensorbitantrioleat (Merck)
3. Nonidet P40: Octyl-phenyl-polyethylenglykol (Fluka)
4. Brij (35, 56, 58 usw.): (Merck)
   - Brij 35: Polyoxyethylenlaurylether
   - Brij 56: Polyoxyethylen-(10)-cetylether
   - Brij 58: Polyoxyethylen-(20)-cetylether
5. Octyl β-Glucoside (Pierce)
6. Octyl β-Thloglucopyranosid (Pierce)
7. SDS: Natriumlauryl-sulfat (Fluka)
8. CHAPS: 3-[(3-Cholamidopropyl)-dimethylammonio]-propansulfonat (Fluka)
9. CHAPSO: 3-[(3-Cholamidopropyl)-dimethylammonio]-2-hydroxypropansulfonat (Fluka)

Bei dem erfindungsgemäßen Verfahren wird eine Elektrodepositionslacksuspension gegebenenfalls mit Wasser verdünnt und mit einer bestimmten Konzentration des Stoffes 4 eingestellt. Das Verhältnis von Emulsion zu gegebenenfalls weiterem Wasser zu Stoff 4 beträgt vorzugsweise 1-10:4-100:0,001-1, bevorzugter 2-5:2-50:0,002-0,04. Die Elektroabscheidung kann in einem beliebigen Gefäß erfolgen. Der Körper (2), in der Regel eine großflächige Elektrode, wird gegebenenfalls vorbehandelt, in die Elektrolytlösung getaucht und ihr gegenüber die Elektrode (5) angeordnet. Zwischen beiden Elektroden wird ein Potential angelegt und gegebenenfalls mit einer Referenzelektrode, beispielsweise einer Kalomelelektrode oder eine Ag/AgCl-Standardelektrode, gesteuert. Die Durchführung der Elektroabscheidung hängt von der Größe der Elektrode bzw. der gewählten Konzentration an Elektrodepositionslackharz sowie von der Verträglichkeit des Enzyms für den Vorgang selbst ab. Möglich sind Potentialpulsprofile von 1500 mV für 1 s, dann 700 mV für 1 s und anschließend 0 mV für 5 s jeweils gegen die Silber/Silberchlorid-Standardelektrode mit jeweils 2 Zyklen. Vor Gebrauch wird die mit Enzym/Harz beschichtete Elektrodenoberfläche bei 4°C über Nacht in 0,1 M Phosphatpuffer, pH 7, oder einem anderen geeigneten Puffer aufbewahrt, um die Elektrode zu konditionieren.

Von der so erhaltenen Elektrode wird eine Kalibrierkurve gegen bekannte Enzymsubstratkonzentrationen aufgenommen. Über einen breiten Konzentrationsbereich ist diese Kurve in der Regel nicht linear, über einen kurzen Konzentrationsbereich kann jedoch Linearität angenähert werden. Nach Erstellung der Kalibrierkurve ist die funktionalisierte Oberfläche gebrauchsfertig, um z.B. unbekannte Konzentrationen über den Erkennungsvorgang zu messen.

Für Kohlenstoffelektroden, die mittels Dickschichttechnik hergestellt wurden, kann eine ähnliche Immobilisierung vorgenommen werden. Die Elektrode wird allerdings nicht vor Gebrauch mit einer Polierpaste behandelt. Das angelegte Potentialpulsprofil kann beispielsweise 2000 mV für 1 s, dann 700 mV für 1 s und anschließend 0 mV für 5 s jeweils gegen Ag/AgCl-Standardelektrode bei 2 Zyklen sein. Die weiteren Vorgehensweisen, wie Aufnahme der Kalibrierkurve und Messungen, erfolgen dann wie vorstehend beschrieben.

Nach Herstellung z.B. einer Enzymelektrode kann diese in gleicher Weise mit einer weiteren Schicht versehen werden (funktionalisierte Flächen mit Schutzschicht). Diese zweite Schicht kann ein weiteres Enzym oder einen katalytischen Stoff oder einen Mediator für Elektronen, Hydroniumionen oder Hydroxylionen enthalten. Z.B. kann auf eine GOD-Schicht, die wie vorstehend beschrieben auf einer Platinelektrode abgeschieden wurde, eine weitere Schicht mit Katalase aufgebracht werden. Die Katalase schützt z.B. vor entstehendem Wasserstoffperoxid. Durch die Katalaseschicht werden thermische und reaktionsaggressive Effekte, die bei Oxidasenreaktionen häufig zu verzeichnen sind, gemildert, unterdrückt oder beseitigt.

Der Aufbau von Mehrschichtsensoren kann wie bei der Herstellung einer Schutzschicht in ähnlicher Weise erfolgen, nur daß hier mehr als zwei verschiedene Schichten übereinander abgeschieden werden. Denkbar sind beispielsweise bestimmte Enzymketten, die Teilsequenzen von biologischen enzymatischen Zyklen erfassen.

Neben der ganzflächigen Abscheidung auf einer Zielelektrode (2) kann durch Einsatz einer punktförmigen Elektrode (5) (Sonde) eine gezielte Abscheidung erfolgen. Dadurch gelingt es, auf einer Fläche unterschiedliche Enzyme nach Wechsel der Abscheidungslösung zu erzielen. Des weiteren ist es möglich, auf einer Fläche verschiedene Enzymkonzentrationen, einmal durch punktförmiges Abtasten der Oberfläche von Elektrode (2), so daß ein Punktraster entsteht, oder durch Anlegen eines Potentialfeldes, wobei ein Kontinuum entsteht (ähnlich der Hullzelle), herzustellen. Mit Hilfe dieser Anordnung ist es möglich, chemisch kombinatorische Untersuchungen vorzunehmen. Der Vorgang ist auch umkehrbar. Abgeschiedene Erkennungs-funktionalisierte Harzflächen lassen sich gezielt mit einer Sonde auflösen, so daß ein Punktraster oder ein Dichtekontinuum entsteht.

Die erfindungsgemäßen Gegenstände können vorzugsweise auf dem Gebiet der Biosensorik der biologischen und chemischen Kombinatorik, der chemischen Diagnostik, beispielsweise Screening, und der Katalysatorforschung eingesetzt werden. Durch den erfindungsgemäßen Gegenstand werden Sensoren auf einfache Weise mit vielfacher Funktion bereitgestellt. Es ist möglich, auf einer Sensoroberfläche verschiedenste Vorgänge gleichzeitig ablaufen zu lassen.

### Beispiele

Eingesetzte Reagenzien:
Acros, New Jersey, USA
   - N-Hydroxy-succinimid 98%
J.T. Baker, Deventer, Holland
   - D(+) Glucose-1-hydrat z.A.
   - Wasser für die HPLC
   - Wasserstoffperoxid 30% z.A.
BASF, Münster, Deutschland
   - Depositionslack ZQ8-43225
Janssen Chimica, Geel, Belgien
   - Lithiumperchlorat z.A.
Merck, Darmstadt, Deutschland
   - Hexachloroplatin(IV)-säure-Hexahydrat zur Synthese
   - Kaliumchlorid Suprapur
Sigma, Deisenhofen, Deutschland
   - EDAC 1-Ethyl-3-(3-dimethylamino-propyl)carbodiimid
   - Glucoseoxidase Typ X-S von Aspergillus Niger 100000-250000 U/g
   - Katalase von Bovine Liver 2100 U/mg
   - Latex Beads 0,431 µm Carboxylat modifiziert und fluoreszent
Strem Chemicals, Newburyport, USA
   - Hexaamminruthenium(III)chlorid

### Beispiel 1

### 1) Glucosesensor:

250 µl EDP-Lacksuspension werden mit 5 ml Wasser (für HPLC) verdünnt. Es wird eine Enzymkonzentration von 3 mg Glucoseoxidase/ml Lösung eingestellt. An eine Platinelektrode (∅ 1 mm) in dieser Lösung wurde folgendes Potentialpulsprofil angelegt (1500 mV für 1 s, dann 700 mV für 1 s und anschließend 0 mV für 5 s jeweils gegen Ag/AgCl. Es wurden 2 Zyklen durchgeführt). Die Elektrode wurde vor ihrem Gebrauch mit 3 µm, 1 µm und 0,3 µm Polierpaste behandelt und anschließend platinisiert. Vor der Aufnahme der Kalibrierkurve wurde sie bei 4°C über Nacht in 0,1 M Phosphatpuffer, pH 7, aufbewahrt.

Die Aufnahme der Kalibrierkurve (Fig. 2) erfolgte in 20 ml Phosphatpuffer, pH 7, durch Zugabe verschiedener Volumina einer 100 mM Glucoselösung. Das dabei an der beschichteten Elektrode angelegte Potential betrug 600 mV gegen Ag/AgCl. Für die mittels Dickschichttechnik hergestellte Kohlenstoffelektrode wurde eine ähnliche Immobilisierungsmethode angewandt, aber mit dem Unterschied, daß die Elektrode vor dem Gebrauch nicht mit Polierpasten behandelt wurde. Das angelegte Potentialpulsprofil lautete wie folgt, 2000 mV für 1 s, dann 700 mV für 1 s und anschließend 0 mV für 5 s jeweils gegen Ag/AgCl. Es wurden 2 Zyklen durchgeführt. Die Aufnahme der Kalibrierkurve (Fig. 3) erfolgte nach dem oben beschriebenen Verfahren.

### Beispiel 2

### Glucosesensor mit Katalase-Schutzschicht

Es wird wie in Beispiel 1 vorgegangen. Anstelle der GOD-Enzymkonzentration von 3 mg/ml Lösung wurden 3 mg Katalase pro ml Lösung eingesetzt. Die verwendeten 1 mm ∅ Platinscheibenelektroden wurden wie unter Beispiel 1 vorbehandelt. Das angelegte Potentialpplsprofil lautete 2000 mV für 1 s, dann 800 mV für 1 s und anschließend 0 mV für 5 s, jeweils gegen Ag/AgCl. Als Vergleichselektrode diente eine in gleicher Weise behandelte Elektrode, welche mit einem GOD-Film modifiziert war (Fig. 4), und eine unmodifizierte (Fig. 5) Platinelektrode.

Die Messung der Wasserstoffperoxid-Sensitivität erfolgte in 20 ml 100 mM Phosphatpuffer, pH 7, durch Zugabe verschiedener Volumina einer 2 mM Wasserstoffperoxidlösung. Das dabei an der beschichteten Elektrode angelegte Potential betrug 600 mV gegen Ag/AgCl.

### Beispiel 3

### Aufbau von Multischichtsensoren (sequentielle Abscheidung):

Es wird wie in Beispiel 1 vorgegangen, wobei eine dünne Schicht gebildet wird. Im Anschluß wird die Abscheidelösung ausgetauscht und der Abscheidevorgang wiederholt.

### Beispiel 4

### Abscheidung auf Sensorarrays:

Es wird wie in Beispiel 1 vorgegangen mit Ausnahme des Polierschritts. Es entsteht eine homogene entsprechend den Abmessungen der leitfähigen Teile der Struktur lateral begrenzte Polymerschicht. (Potentialpulsprofil: 2000 mV für 0,5 s// 700 mV für 1 s// 0 mV für 5 s// ein Zyklus). Die Abscheidung wurde auf einer Mikrobandstruktur (1000 µm * 20 µm) in einer GOD-haltigen Lösung durchgeführt, vgl. auch Fig. 11.

### Beispiel 5

### Einschluß von fluoreszierenden Beads

Es wird wie in Beispiel 4 vorgegangen. Es entsteht ein Polymerfilm, welcher auf die Abmessungen der beschichteten Mikrostruktur beschränkt ist. Die Polymerlösung enthält 250 µl EPD-Lack in 5 ml Wasser, welches mit ca. 10 µl der Beadsuspension versetzt wurde. (Potentialpulsprofil: 2000 mV für 1 s// 800 mV für 1 s// 0 mV für 5 s// ein Zyklus). Bei der beschichteten Mikrostruktur handelt es sich um ein Dreielektrodensystem. Die erste Figur (Fig. 6) zeigt die Mikrostruktur mit zwei beschichteten Mikrobandelektroden bei Normallicht. Die L-förmige Bandelektrode wurde mit einem GOD-Film beschichtet (dasselbe Potentialpulsprofil wie für die Beadabscheidung), während die kleinere, gerade Bandelektrode mit dem Beadfilm versehen wurde. Bei der mittleren Mikrobandelektrode handelt es sich um eine Ag/AgCl-Referenzelektrode.

Vergleichend zu der obigen Figur läßt sich auf der mittels eines Fluoreszenzmikroskops gemachten Aufnahme (Fig. 6) derselben Mikrostruktur nur noch der mit fluoreszierenden Latex-Beads versetzte Film erkennen, welcher selektiv auf der Bandelektrode abgeschieden wurde.

Die Latex-Beads dienten in diesem Fall als Testsubstanz, um die Möglichkeit für den Einschluß von größeren und kleineren Partikeln in den Film zu überprüfen. Denkbar ist der Einschluß von mit Erkennungselementen oder Ankergruppen modifizierten Partikeln (Beads), um so auf einfache Art und Weise sogenannte "Screening-Assays" herzustellen. Diese könnten dann mittels unterschiedlicher Detektionsmethoden, wie z.B. der Fluoreszenz, ausgewertet werden. Eine weitere sich bietende Anwendung wäre der Aufbau von kombinatorischen Bibliotheken.

### Beispiel 6

### Mikrostrukturierte (lokale) Abscheidung auf leitenden Oberflächen (nicht manuelle Adressierbarkeit):

Die mikrostrukturierte Abscheidung des Polymers auf leitenden Oberflächen wurde mittels der elektrochemischen Rastersondenmikroskopie durchgeführt. Bei dieser Methode wird eine (Nano-) Mikroelektrode mittels eines positiven oder negativen Feedbacks, welcher auf einer Veränderung des in einer Redoxspezies enthaltenden Elektrolytlösung (5 mM Rutheniumhexaammin-Lösung) gemessenen Faraday'schen Stroms an der Mikroelektrode basiert, an die Oberfläche angenähert (5-10 µm) [C. Kranz, M. Ludwig, H.E. Gaub, W. Schuhmann, Adv. Materials, 7 (1995) 568-571]. Anschließend wird die Elektrolytlösung gegen die Polymerlösung (GOD-Polymerlösung mit 0,5 mg KCl) ausgetauscht, die leitende Oberfläche als Arbeitselektrode und die angenäherte Mikroelektrode als Gegenelektrode geschaltet. Als Mikroelektrode wurden für die Versuche neben glasummantelten Platinscheibenelektroden auch Carbonfaser-Mikroelektroden (Carbonfaser ∅ 8 µm, Sigrafil, isoliert mit EDP-Lack (Electrodeposition Paint)) eingesetzt. Die leitende Oberfläche bestand aus einer Scheibengoldelektrode (∅ 3 mm).

Nun kann in dem Volumen zwischen Arbeits- und Gegenelektrode durch Veränderung des Potentials der pH-Wert lokal geändert werden. Dieses führt dann zu einer lokalen Ausfällung des Polymers unterhalb der Mikroelektrode. Das gewählte Potentialpulsprofil lautete zuerst 800 mV für 1 s und anschließend 0 mV für 10 s, jeweils gegen Ag/AgCl. Dieses Pulsprofil wurde 5mal wiederholt. Durch die Veränderung des Anfangspulses und der Zyklenzahl wurden unterschiedlich dichte und große Filme hergestellt (Fig. 7). Die entstandenen Polymerpunkte auf der Goldoberfläche hatten einen Durchmesser von 50 bis 100 µm.

### Beispiel 7

### Lokale Auflösung des Polymerfilms (nicht manuelle Auflösung)

Bei der lokalen Auflösung des Polymerfilms wird durch eine lokal erzeugte Änderung des pH-Werts der Film selektiv an dieser Stelle aufgelöst. Anschließend kann durch einen Spülvorgang das aufgelöste Polymer entfernt werden. Die wieder zugänglichen Stellen der Oberfläche können nun anschließend wieder funktionalisiert werden, vgl. Fig. 8.

Für die lokale Auflösung des Films wurde zuerst eine Goldscheiben-elektrode (∅ 3 mm) mit dem Polymerfilm vollständig beschichtet. Anschließend wurde eine glasummantelte Platinscheibenelektrode (∅ 50 µm) an die Filmoberfläche angenähert (wie unter Beispiel 6). Nun wurde ein Potential an der Mikroelektrode angelegt, welches zur kathodischen Zersetzung von Wasser und somit zur Entstehung von OH- unterhalb der Mikroelektrode führt. Durch diese pH-Wert-Änderung kann der Film lokal aufgelöst werden.

Für die Auflösung wurde ein Potentialpuls von -2000 mV gegen Ag/AgCl für jeweils 1 s an der Mikroelektrode angelegt und anschließend mehrfach wiederholt. Das im Bild (Fig. 8) zu sehende L ist ein an dieser Stelle teilweise aufgelöster Film, es entstand durch eine laterale Verschiebung der Elektrode während des Pulsvorganges. Vergleichend dazu ist noch ein Kratzer auf die Filmoberfläche gemacht worden.

### Beispiel 8

### Modifizierung der Polymerfilme

Da die EDPs funktionelle Gruppen, wie Säure- oder Aminogruppen, aufweisen, besteht eine gute Möglichkeit für ihre kovalente Modifizierung. Die Modifizierung kann je nach den Erfordernissen sowohl vor der Abscheidung als auch nach der Abscheidung erfolgen. Auch bei der Art der einzusetzenden Modifizierungsmittel ist man nur von deren Stabilität in der Polymerlösung abhängig. Der Film wurde z.B. mit redoxaktiven Gruppen modifiziert.

Dafür wurde zu einer Suspension, bestehend aus 150 µl Lack und 3 ml Phosphatpuffer (0,1 M und pH 7), 0,5 mM EDAC und 20 mM N-Hydroxy-succinimid, dazugegeben. Dieses wurde nun für 3 h bei Raumtemperatur gut geschüttelt und anschließend mittels der Elektrodeposition abgeschieden.

Potentialpulsprofil: 2000 mV für 0,2 s// 700 mV für 1 s// 0 mV für 5 s// 5 Zyklen. Die so modifizierte Elektrode wurde für 24 h bei Raumtemperatur in eine 10 mM [Os(bpy)₂(Histamin)Cl]Cl-Lösung getaucht. Anschließend wurde die Elektrode gründlichst mit Wasser gespült und für mehrere Stunden in Wasser aufbewahrt. Nach diesem Schritt wurde von dieser Elektrode mehrere CV's (Fig. 9) und DPV's (Fig. 10) in 0,1 M Lithiumperchlorat-Lösung aufgenommen. Wie auf den beiden Bildern zu erkennen, sind auf der Elektrode redoxaktive Komponenten (der Osmiumkomplex) vorhanden.

## Patentansprüche

1. Verfahren zur Herstellung einer mit Erkennungselementen funktionalisierten Fläche (1), umfassend einen gegebenenfalls elektrisch leitfähigen Körper (2) und darauf aufgebracht, mindestens einen Stoff (4) mit Erkennungsfunktion enthaltende Harzschicht (3), **dadurch gekennzeichnet, daß**
a) Harzschicht (3) durch Elektrodeposition auf Körper (2) abgeschieden wird,
b) Harzschicht (3) von polymerisierbaren, ionentragenden Monomeren abgeleitete Harze umfaßt, die durch Änderung des pH-Werts abgeschieden werden und
c) der oder die Stoffe (4) und gegebenenfalls weitere Zusätze während der Elektrodeposition mit dem Harz aus einer Harzemulsion auf Körper (2) abgeschieden wird/werden.

2. Verfahren nach Anspruch 1, wobei das Harz der Harzschicht (3) von Acrylmonomeren abgeleitet ist.

3. Verfahren nach Anspruch 1 oder 2, wobei die Elektrodeposition durch Erzeugung von H⁺-lonen auf Körper (2) erfolgt.

4. Verfahren nach Anspruch 1 oder 2, wobei die Elektrodeposition durch Erzeugung von OH⁻-lonen auf Körper (2) erfolgt.

5. Verfahren nach einem der Ansprüche 1 bis 3, wobei der zur Elektrodeposition verwendete Elektrolyt eine Emulsion aus Wasser und einer Harzzubereitung auf der Basis eines Carboxylgruppen, gegebenenfalls auch verkappte Isocyanatgruppen, Hydroxyl- und/oder Ethergruppen enthaltenden Copolymerisats, das durch zumindest teilweise Salzbildung mit Ammoniak oder einer organischen oder anorganischen Base wasserlöslich oder in Wasser dispergierbar ist, umfaßt.

6. Verfahren nach einem der Ansprüche 1, 2 oder 4, wobei der zur Elektrodeposition verwendete Elektrolyt eine Emulsion aus Wasser und einer Harzzubereitung auf der Basis eines organische Aminogruppen aufweisenden Monomers, Oligomers oder Polymers umfaßt, wobei die Harzzubereitung basischer Beschaffenheit ist und die Aminogruppen gegebenenfalls mit C₁-C₆-Alkylgruppen substituiert sind, die ihrerseits gegebenenfalls mit OH oder gegebenenfalls substituierten Aminogruppen substituiert, und/oder durch Heteroatome, ausgewählt aus S, O, NR₁, unterbrochen sind, wobei R₁ eine C₁-C₆-Alkylgruppe bedeutet.

7. Verfahren nach einem der Ansprüche 1-6, wobei als gegebenenfalls elektrisch leitfähiger Körper (2) ein Edelmetall, ausgewählt aus Gold, Silber, Platin, Palladium, Iridium, Rhenium, Quecksilber, Ruthenium und Osmium, vorzugsweise Platin, verwendet wird.

8. Verfahren nach einem der Ansprüche 1-7, wobei mindestens zwei Elektrodepositionen mit unterschiedlichen Stoffen (4) auf Körper (2) abgeschieden werden.

9. Verfahren nach einem der Ansprüche 1-8, wobei die Abscheidung auf einem Array erfolgt.

10. Verfahren nach einem der Ansprüche 1-9, wobei die Abscheidung mittels Rastersondenmikroskopie erfolgt, wobei Harzpunkte mit Erkennungselementen gegebenenfalls unterschiedlicher Dichte und Größe erzielt werden.

11. Verfahren zur Herstellung einer mit Erkennungselementen funktionalisierten Fläche nach Anspruch 1, wobei zunächst eine Abscheidung nach einem der Ansprüche 1-10 erfolgt und anschließend diese gezielt unter Ausbildung von Harzflächen mit Erkennungselementen gegebenenfalls unterschiedlicher Dichte und Größe kathodisch oder anodisch aufgelöst wird.

12. Verfahren nach einem der Ansprüche 1-11, wobei Stoff (4) mit Erkennungsfunktion aus Latexbeads, Glasbeads, Graphitteilchen, färbenden, fluoreszierenden, phosphoreszierenden, magnetischen und/oder radioaktiven Teilchen und/oder Teilchen mit chemischen, insbesondere elektrochemischen Eigenschaften, wie Amperometrie, Impedimetrie, Voltammetrie, Redoxvorgänge etc., biologischer und/oder biochemischer Funktion ausgewählt ist.

13. Verfahren nach Anspruch 12, wobei Stoff (4) Teilchen mit biologischer und/oder biochemischer Funktion bedeutet.

14. Verfahren nach Anspruch 13, wobei Stoff (4) aus mindestens einem von Enzymen, Antikörpern, Antigenen, Haptenen, DNA, RNA, Oligonucleotiden, Peptiden, Oligopeptiden, Proteinen, Lectinen, Hormonen, Rezeptorantagonisten oder -Agonisten, Zellen und Mikroorganismen ausgewählt ist.

15. Verfahren nach Anspruch 13 oder 14, wobei Stoff (4) ein Enzym ist.

16. Verfahren nach Anspruch 12, wobei der Stoff (4) ein anorganischer Katalysator ist, ausgewählt aus Cu/ZnO, Au/TiO₂, Na⁺ dotierten Übergangsmetallen und/oder Metallen der VIII. Nebengruppe.

17. Verfahren nach einem der Ansprüche 1-16, wobei durch Änderung der Verfahrensparameter, insbesondere der Höhe des elektrischen Potentials, der Diffusion, der Kondensation und/oder der Schichtdicke des Elektrolyten eine Abscheidung unterschiedlicher Dicke und/oder Dichte erzielt wird.

## Claims

1. Method for producing a surface (1) functionalized by detection elements, comprising an optionally electrically conductive body (2) and a resin layer (3) containing at least one substance (4) with a detection function applied thereto, **characterized in that**
a) the resin layer (3) is deposited on the body (2) by electro-deposition,
b) the resin layer (3) comprises resins derived from polymerisable, ion-carrying monomers which are deposited by changing the pH value and
c) the substance(s) and optionally further additives is/are deposited with the resin from a resin emulsion on the body (2) during the electro-deposition.

2. Method according to claim 1, wherein the resin of the resin layer (3) is derived from acrylic monomers.

3. Method according to claim 1 or 2, wherein the electro-deposition is effected by creation of H⁺ ions on the body (2).

4. Method according to claim 1 or 2, wherein the electro-deposition is effected by creation of OH⁻ ions on the body (2).

5. Method according to any of claims 1 to 3, wherein the electrolyte used for the electro-deposition comprises an emulsion of water and a resin preparation on the basis of a copolymerisate which contains carboxyl groups, optionally also masked isocyanate groups, hydroxyl and/or ether groups and which is soluble or dispersible in water through at least partial salt formation with ammonia or an organic or inorganic base.

6. Method according to any of claims 1, 2 and 4, wherein the electrolyte used for the electro-deposition comprises an emulsion of water and a resin preparation on the basis of a monomer, oligomer or polymer having organic amino groups, wherein the resin preparation is of basic nature and the amino groups are optionally substituted with C₁-C₆ alkyl groups, which for their part are optionally substituted with OH or optionally substituted amino groups, and/or are interrupted by heteroatoms, selected from S, O, NR₁, wherein R₁ signifies a C₁-C₆ alkyl group.

7. Method according to any of claims 1 to 6, wherein as an optionally electrically conductive body (2) there is used a noble metal selected from gold, silver, platinum, palladium, iridium, rhenium, mercury, ruthenium and osmium, preferably platinum.

8. Method according to any of claims 1 to 7, wherein at least two electrodeposits with different substances (4) are deposited with on the body (2).

9. Method according to any of claims 1 to 8, wherein the deposition is effected in an array.

10. Method according to any of claims 1 to 9, wherein the deposition is effected by means of raster probe microscopy, wherein resin points with detection elements optionally of different densities and sizes are created.

11. Method of producing a surface functionalized with detection elements according to claim 1, wherein a deposition according to any of claims 1 - 10 is initially effected and then this is selectively cathodically or anodically dissolved to form resin areas with detection elements, optionally of different densities and sizes.

12. Method according to any of claims 1 to 11, wherein a substance (4) with a detection function is selected from latex beads, glass beads, graphite particles, colouring, fluorescing, phosphorescing, magnetic and/or radioactive particles and/or particles with chemical, especially electrochemical properties, such as amperometry, impedimetry, voltametry, redox processes, etc., a biological and/or biochemical function.

13. Method according to claim 12, wherein substance (4) signifies particles with a biological and/or biochemical function.

14. Method according to claim 13, wherein a substance (4) is selected from at least one of enzymes, antibodies, antigens, haptens, DNA, RNA, oligonucleotides, peptides, oligopeptides, proteins, lectins, hormones, receptor antagonists or agonists, cells and microorganisms.

15. Method according to claim 13 or 14, wherein substance (4) is an enzyme.

16. Method according to claim 12, wherein the substance (4) is an inorganic catalyst, selected from Cu/ZnO, Au/TiO₂, transition metals doped with Na⁺ and/or metals of the VIII transition group.

17. Method according to any of claims 1 to 16, wherein deposition of different thickness and/or density is achieved by altering the process parameters, especially the level of the electric potential, the diffusion, the condensation and/or the layer thickness of the electrolytes.

## Revendications

1. Procédé pour réaliser une surface (1) rendue fonctionnelle avec des éléments de reconnaissance, comprenant un corps (2), en option électriquement conducteur, et au moins une couche de résine (3) appliquée sur ce corps contenant au moins un produit (4) avec fonction de reconnaissance, **caractérisé en ce que**
a) on dépose la couche de résine (3) sur le corps (2) par électrodéposition,
b) la couche de résine (3) contient des résines polymérisables dérivées de monomères porteur d'ions, qui sont déposées par modification de la valeur du pH, et
c) le ou les produits (4) et le cas échéant d'autres additifs est/sont déposé(s) sur le corps (2) pendant l'électrodéposition avec la résine à partir d'une émulsion de résine.

2. Procédé selon la revendication 1, dans lequel la résine de la couche de résine (3) est dérivée de monomères acryliques.

3. Procédé selon la revendication 1 ou 2, dans lequel l'électrodéposition a lieu par génération d'ions H⁺ sur le corps (2).

4. Procédé selon la revendication 1 ou 2, dans lequel l'électrodéposition a lieu par génération d'ions OH⁻ sur le corps (2).

5. Procédé selon l'une des revendications 1 à 3, dans lequel l'électrolyte utilisé pour l'électrodéposition comprend une émulsion d'eau et d'une préparation de résine sur la base d'un copolymérisat contenant des groupes carboxyle, le cas échéant également des groupes isocyanates masqués, des groupes hydroxyle et/ou éther, qui est soluble dans l'eau ou susceptible d'être dispersé dans l'eau par formation au moins partielle de sel avec de l'ammoniaque ou avec une base organique ou non organique.

6. Procédé selon l'une des revendications 1, 2 ou 4, dans lequel l'électrolyte utilisé pour l'électrodéposition comprend une émulsion d'eau et une préparation de résine sur la base d'un monomère, d'un oligomère ou d'un polymère présentant des groupes aminés organiques, ladite préparation de résine ayant des propriétés basiques, et les groupes aminés sont éventuellement substitués par des groupes alkyle C₁-C₆- qui sont à leur tour éventuellement substitués avec OH ou des groupes aminés éventuellement substitués, et/ou sont interrompus par des hétéro-atomes, choisis parmi S, O, NR₁, R₁ signifiant un groupe alkyle C₁-C₆-.

7. Procédé selon l'une des revendications 1 à 6, dans lequel on utilise comme corps (2) éventuellement électroconducteur un métal précieux, choisi parmi or, argent, platine, palladium, iridium, rhénium, mercure, ruthénium et osmium, de préférence platine.

8. Procédé selon l'une des revendications 1 à 7, dans lequel on procède à au moins deux électrodépositions sur le corps (2) avec des produits différents (4).

9. Procédé selon l'une des revendications 1 à 8, dans lequel la déposition a lieu sur une série.

10. Procédé selon l'une des revendications 1 à 9, dans lequel la déposition a lieu au moyen de microscopie à sonde tramée, de sorte que l'on obtient des points de résine avec des éléments de reconnaissance présentant éventuellement une densité et une taille différentes.

11. Procédé pour réaliser une surface rendue fonctionnelle, selon la revendication 1, dans lequel a lieu tout d'abord une déposition selon l'une des revendications 1 à 10, et celle-ci est ensuite dissoute de façon ciblée par voie cathodique ou anodique en réalisant des surfaces de résine avec des éléments de reconnaissance présentant éventuellement une densité et une taille différentes.

12. Procédé selon l'une des revendications 1 à 11, dans lequel le produit (4) présentant une fonction de reconnaissance est choisi parmi des billes de latex, des billes de verre, des particules de graphite, des particules colorantes, fluorescentes, phosphorescentes, magnétiques et/ou radioactives et/ou des particules présentant des propriétés chimiques, en particulier électrochimiques, comme ampérométrie, impédimétrie, voltamétrie, processus redox, etc., avec fonction biologique et/ou biochimique.

13. Procédé selon la revendication 12, dans lequel le produit (4) est constitué de particules avec fonction biologique et/ou biochimique.

14. Procédé selon la revendication 13, dans lequel le produit (4) est choisi parmi au moins un des éléments du groupe enzymes, anticorps, antigènes, haptènes, ADN, ARN, oligo-nucléotides, peptides, oligopeptides, protéines, lectines, hormones, récepteurs antagonistes ou agonistes, cellules et micro-organismes.

15. Procédé selon la revendication 13 ou 14, dans lequel le produit (4) est un enzyme.

16. Procédé selon la revendication 12, dans lequel le produit (4) est un catalyseur inorganique, choisi parmi des métaux de transition dopés avec Cu/ZnO, Au/TiO₂, Na⁺, et/ou des métaux de transition du sous-groupe VIII.

17. Procédé selon l'une des revendications 1 à 16, dans lequel, par modification des paramètres du procédé, en particulier l'intensité du potentiel électrique, de la diffusion, de la condensation et/ou de l'épaisseur de couche de l'électrolyte, on obtient une déposition d'épaisseur et/ou de densité différentes.
